# EUROPÄISCHE PATENTSCHRIFT

(11) **EP 4 507 655 B1**
(45) Veröffentlichungstag und Bekanntmachung des Hinweises auf die Patenterteilung: **14.10.2026**
(21) Anmeldenummer: 23700662.2
(22) Anmeldetag: 10.01.2023
(51) Int. Cl.: A61K 8/06, A61K 8/19, A61K 8/58, A61K 8/60, A61K 8/86, A61K 8/895, A61K 8/898, A61Q 5/06, A61Q 5/12, A61K 8/04

(54) **VERFAHREN ZUR FÄRBUNG VON KERATINFASERN, UMFASSEND DIE ANWENDUNG EINES FÄRBEMITTELS MIT AMINOSILIKON UND PIGMENT UND EINES CONDITIONERS MIT PFLEGESTOFF**
METHOD FOR COLOURING KERATIN FIBRES COMPRISING THE APPLICATION OF A COLOURANT WITH AMINO SILICONE AND PIGMENT AND A CONDITIONER WITH CONDITIONING AGENT
PROCEDE DE COLORATION DES FIBRES KERATINIQUES, COMPRENANT L'APPLICATION D'UN AGENT DE COLORATION COMPRENANT UNE AMINOSILICONE ET UN PIGMENT, ET D'UN CONDITIONNEUR COMPRENANT UN AGENT DE SOIN

(30) Priorität: 12.04.2022 DE 102022203642
(43) Veröffentlichungstag der Anmeldung: 19.02.2025
(73) Patentinhaber: Henkel AG & Co. KGaA, 40589 Düsseldorf (DE)
(72) Erfinder: KRUCK, Constanze, 41515 Grevenbroich (DE); WESER, Gabriele, 45219 Essen (DE); KOLONKO, Claudia, 42857 Remscheid (DE); BREUER, Imme, 40474 Düsseldorf (DE)
(86) Internationale Anmeldenummer: PCT/EP2023/050411
(87) Internationale Veröffentlichungsnummer: WO 2023/198316

(56) Entgegenhaltungen:
- WO-A1-2005/058259
- WO-A1-2016/133811
- WO-A2-2012/079873
- DE-A1- 10 058 328

## Beschreibung

Gegenstand der vorliegenden Anmeldung ist ein Verfahren zum Färben von keratinischen Fasern, insbesondere menschlichen Haaren, welches die Anwendung eines Färbemittels (F) und die Anwendung eines Conditioners (C) umfasst. Hierbei enthält das Färbemittel (F) mindestens ein aminofunktionalisiertes Silikonpolymer (f1) und mindestens ein Pigment (f2). Der Conditioner (C) enthält mindestens einen Pflegestoff aus einer bestimmten Gruppe.

Die Veränderung von Form und Farbe von keratinischen Fasern, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Pigmenten oder Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Ein mögliches, alternatives Färbesystem, das in letzter Zeit zunehmend in den Fokus rückt, beruht auf dem Einsatz von farbigen Pigmenten.

Die Färbung mit Pigmenten bietet verschiedene wesentliche Vorteile. Da die Pigmente sich lediglich von außen an die Keratinfasern, insbesondere an die Haarfasern, anlagern, ist die mit dem Färbeprozess verbundene Schädigung ganz besonders gering. Weiterhin lassen sich nicht mehr erwünschte Färbungen schnell und einfach rückstandslos entfernen und bieten dem Anwender auf diese Weise die Möglichkeit, unmittelbar und ohne großen Aufwand zu seiner Ursprungshaarfarbe zurück zu kehren. Insbesondere für die Konsumenten, die ihre Haare nicht regelmäßig nachfärben möchten, ist dieser Färbeprozess daher besonders attraktiv.

In aktuellen Arbeiten wurde das Problem der geringen Haltbarkeit dieses Färbesystems adressiert. In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte. Darüber hinaus konnte durch die Wahl besonders gut geeigneter Pigmente und Pigmentkonzentrationen auf dunklem Haar ein helleres Farbergebnis erzielt werden, so dass mit diesem Färbesystem sogar eine Aufhellung möglich wurde, die bis dato ausschließlich mit oxidativen Haarbehandlungsmitteln (Bleich- bzw. Blondiermitteln) möglich war.

Neben diesen vielen Vorteilen besitzt das auf Pigmenten basierende Färbesystem jedoch immer noch einige Nachteile. Da sowohl die Pigmente als auch die Aminosilikone, welche die Pigmente immobilisieren, sich auf der Oberfläche der Haarfaser ablagern, wird deren Oberflächenstruktur durch Ausbildung eines Films modifiziert. Abhängig von der Dicke des ausgebildeten Films kann mit dieser Modifikation auch eine Veränderung des haptischen Eindrucks der Haarfasern verbunden sein, der von dem Gefühl, beschwerte oder fettige Haare zu haben, bis zu einem rauen, struppigen oder strohigen Griff der Haare reicht.

Es war die Aufgabe der vorliegenden Erfindung, ein auf Pigmenten basierendes Färbesystem bereitzustellen, das intensive Farbergebnisse bei gutem Haargefühl ermöglicht. Es wurde nach einer Technologie gesucht, die es ermöglicht, farbige Pigmente möglichst dauerhaft auf den Haaren zu fixieren, ohne dass die Haare sich beschwert, fettig, unnatürlich, strohig, belegt oder klebrig anfühlen. Ein besonderer Fokus der Aufgabenstellung lag auf der Erzielung von intensiven Farbergebnissen bei gleichzeitig gutem Haargefühl.

Überraschenderweise hat sich nun herausgestellt, dass keratinische Fasern, die einem ersten Schritt durch Anwendung eines Färbmittels mit Aminosilikon und Pigment gefärbt wurden, besonders dann eine Verbesserung im Hinblick ihres Haargefühls erfahren, wenn sie im Folge-schritt mit einem Conditioner behandelt werden, der mindestens einen speziellen Pflegestoff enthält, der ausgewählt ist aus der Gruppe aus Esterquat-Tensiden, Amidoamin-Tensiden, quartären Imidazolin-Tensiden, Homo- oder Co-polymeren des Diallyldimethylammoniumchlorids, quaternisierten Cellulose-Derivaten, Chitosan und Derivaten des Chitosans. Das Färbemittel und der Conditioner werden sukzessive auf die Keratinfasern appliziert und stellen verschiedene Formulierungen dar.

Ein erster Gegenstand der vorliegenden Erfindung ist ein Verfahren zum Färben von keratinischen Fasern, insbesondere menschlichen Haaren, umfassend die
(1) Anwendung eines Färbemittels (F) auf den keratinischen Fasern, welches enthält (f1) mindestens ein aminofunktionalisiertes Silikonpolymer, und (f2) mindestens ein Pigment, welches bei 25 °C in Wasser eine Löslichkeit von weniger als 0,05 g/L besitzt, und die
(2) Anwendung eines Conditioners (C) auf den keratinischen Fasern, welcher enthält (c1) mindestens einen Pflegestoff ausgewählt aus der Gruppe aus Esterquat-Tensiden, Amidoamin-Tensiden, quartären Imidazolin-Tensiden, Homopolymeren des Diallyldimethylammonium-chlorids, Copolymeren des Diallyldimethylammoniumchlorids,quaternisierten Cellulose-Derivaten, Chitosan und Derivaten des Chitosans,
wobei es sich bei dem Färbemittel (F) und bei dem Conditioner (C) um verschiedene Formulierungen handelt, und wobei zwischen den Schritten (1) und (2) ein Zeitraum von maximal 2 Stunden liegt.

Die zu dieser Erfindung führenden Arbeiten haben gezeigt, dass Haare, durch Anwendung von eines Färbemittel (F) enthaltend das Aminosilikon (f1) und Pigment (f2) intensiv gefärbt werden konnten. Das Haargefühl der auf diese Weise gefärbten Haare konnte massiv verbessert werden, wenn nach der Färbung ein Conditioner (C) auf die Keratinfasern appliziert wurde, welcher mindestens einen Pflege-Stoff der oben genannten speziellen Gruppe enthielt. Hierbei war insbesondere überraschend, dass die Anwesenheit eines oder mehrere Pflegestoffe aus dieser Gruppe im Conditioner die Verbesserung des Haargefühls ohne Einbußen im Hinblick auf die Farbintensität ermöglichte.

### Keratinische Fasern

Unter keratinischen Fasern sind Wolle, Pelze und Federn und insbesondere menschliche Haare zu verstehen. Ganz besonders bevorzugt wird unter keratinischen Fasern das menschliche Haar verstanden.

### Mittel zur Färbung

Der Begriff "Mittel zur Färbung" wird im Rahmen dieser Erfindung für eine durch Einsatz von Pigmenten hervorgerufene Farbgebung des Keratinmaterials, insbesondere des Haares, verwendet. Bei dieser Färbung lagern sich die Pigmente als farbgebende Verbindungen zusammen mit dem oder den aminofunktionalisierten Silikonpolymeren in einem besonders homogenen, gleichmäßigen und glatten Film an der Oberfläche des Keratinmaterials ab.

### Verbesserung des Griffgefühls

Unter einer Verbesserung des Griffgefühls wird verstanden, dass die gefärbten Keratinfasern, insbesondere menschliche Haare, beim Griff in die Haare einen natürlicheren und weicheren Eindruck hinterlassen, und dass die gefärbten Keratinfasern bzw. die gefärbten Haare sich weniger beschwert, fettig, ölig, unnatürlich, strohig, rauh oder belegt anfühlen. Auch lassen sich einzelne Haarfasern besser voneinander separieren und kleben nicht zusammen.

Das Griffgefühl ist ein sensorischer Eindruck, der von geübten Personen wie beispielsweise von Frisören erfühlt werden kann. Die Verbesserung des Griffgefühls einer Haarsträhne kann beispielsweise von einem Frisör oder auch von einem erfahrenen Anwender festgestellt werden, indem er zwei verschiedene Strähnen befühlt oder bestastet und so im Vergleich feststellt, welche der beiden Strähnen sich besser anfühlt, d.h. welche der beiden Strähnen einen weniger fettigen, öligen, klebrigen, belegten, rauhen oder strohigen Eindruck macht.

### Anwendung des Färbemittels (F) auf keratinische Fasern

In Schritt (1) des erfindungsgemäßen Färbeverfahrens wird ein Färbemittel (F) auf den keratinischen Fasern bzw. dem menschlichen Haar angewendet, welches mindestens ein aminofunktionalisiertes Silikonpolymer (f1) und mindestens ein Pigment (f2) enthält. Bei dem Färbemittel (F) handelt es sich um ein anwendungsbereites Färbemittel.

### aminofunktionalisiertes Silikonpolymer (f1) im Färbemittel

Das Färbemittel (F) enthält mindestens ein aminofunktionalisiertes Silikonpolymer (f1). Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird auch durch die Polymerisationsmethode mitbestimmt. Im Sinne der vorliegenden Erfindung ist es bevorzugt, wenn das maximale Molekulargewicht des Silikonpolymers nicht mehr als 10⁷ g/mol, bevorzugt nicht mehr als 10⁶ g/mol und besonders bevorzugt nicht mehr als 10⁵ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnte eine gute Färbeleistung mit aminofunktionalisierten Silikonpolymeren erzielt werden, wenn diese mindestens eine primäre, mindestens eine sekundäre und/oder mindestens eine tertiäre Aminogruppe tragen. Intensive Färbungen mit der besten Waschechtheit wurden jedoch erhalten, wenn ein aminofunktionalisiertes Silikonpolymer im Mittel eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass ein Färbemittel (F) auf den keratinischen Fasern angewendet wird, welches mindestens ein aminofunktionalisierten Silikonpolymer (f1) mit mindestens einer sekundären Aminogruppe umfasst.

Die sekundäre(n) Aminogruppe(n) kann bzw. können sich an verschiedenen Positionen des aminofunktionalisierten Silikonpolymers befinden. Ganz besonders gute Farbresultate wurden erhalten, wenn ein aminofunktionalisiertes Silikonpolymer eingesetzt wurde, dass mindestens eine, bevorzugt mehrere Struktureinheiten der Formel (Si-Amino) besitzt.

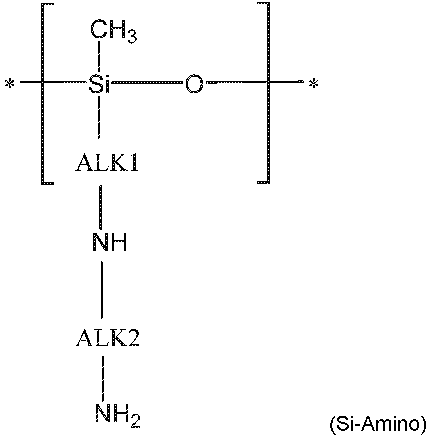

In den Struktureinheiten der Formel (Si-Amino) stehen die Kürzel ALK1 und ALK2 unabhängig voneinander für eine lineare oder verzweigte, zweiwertige C₁-C₂₀-Alkylengruppe.

In einer weiteren ganz besonders bevorzugten Ausführungsform ist ein erfindungsgemäßes Verfahren dadurch gekennzeichnet, dass das Färbemittel (F) mindestens ein aminofunktionalisiertes Silikonpolymer (f1) enthält, das mindestens eine Struktureinheit der Formel (Si-Amino) umfasst, 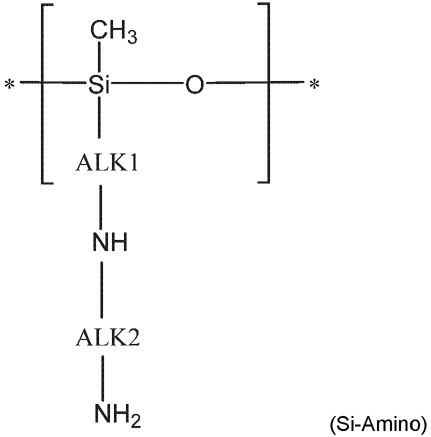 wobei
ALK1 und ALK2 unabhängig voneinander für eine lineare oder verzweigte, zweiwertige C₁-C₂₀-Alkylengruppe stehen.

Die mit einem Stern (*) gekennzeichneten Positionen geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine C₁-C₆-Alkylgruppe gebunden sein.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In einer weiteren besonders bevorzugten Ausführungsform stellen die Struktureinheiten der Formel (Si-Amino) Wiederholungseinheiten im aminofunktionalisierten Silikonpolymer dar, so dass das Silikonpolymer mehrere Struktureinheiten der Formel (Si-Amino) umfasst.

Im Folgenden werden besonders gut geeignete aminofunktionalisierte Silikonpolymere mit mindestens einer sekundären Aminogruppe aufgelistet.

Färbungen mit den allerbesten Waschechtheiten konnten erhalten werden, wenn bei der Färbung mindestens ein Mittel auf dem keratinischen Material appliziert wurde, das mindestens ein aminofunktionalisiertes Silikonpolymer enthält, das Struktureinheiten der Formel (Si-I) und der Formel (Si-II) umfasst

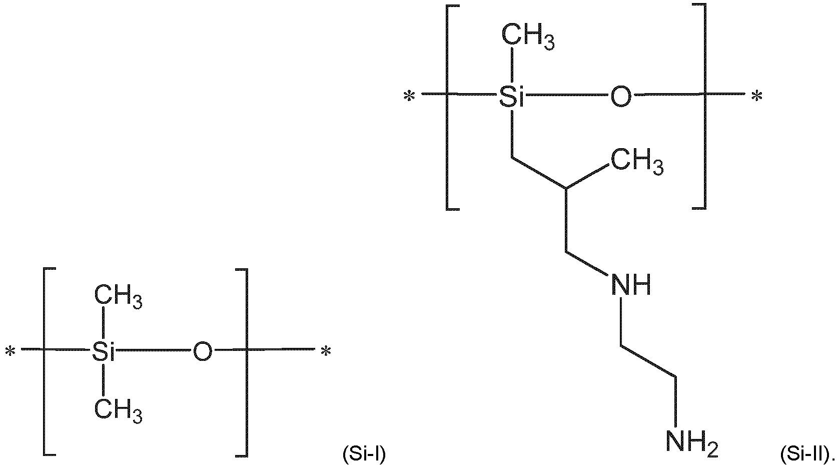

In einer weiteren explizit ganz besonders bevorzugten Ausführungsform ist ein erfindungsgemäßes Verfahren dadurch gekennzeichnet, dass das Färbemittel (F) mindestens ein aminofunktionalisiertes Silikonpolymer (f1) enthält, das Struktureinheiten der Formel (Si-I) und der Formel (Si-II) umfasst

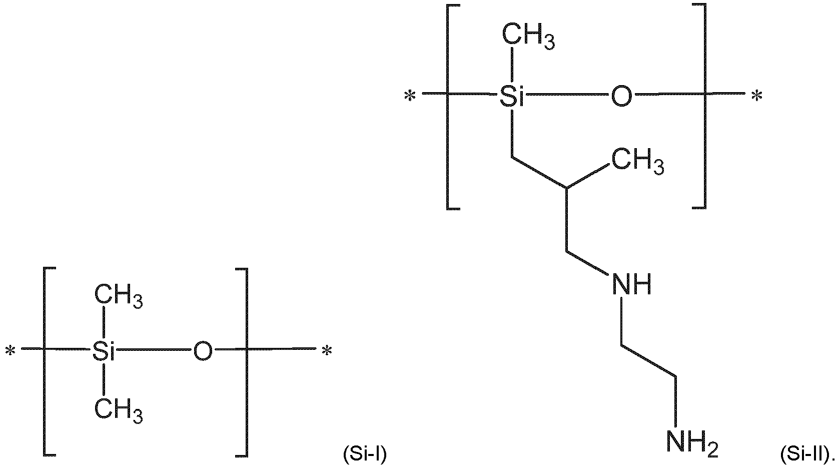

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. Ein weiteres besonders bevorzugtes Handelsprodukt ist Dowsil AP-8568 Amino Fluid, das ebenfalls von der Firma Dow Chemical Company kommerziell vertrieben wird.

Im Rahmen einer weiteren Ausführungsform kann die Färbung auch durch Anwendung eines Färbemittels (F) erfolgen, das mindestens ein aminofunktionelles Silikonpolymer der Formel der Formel (Si-III) enthält, 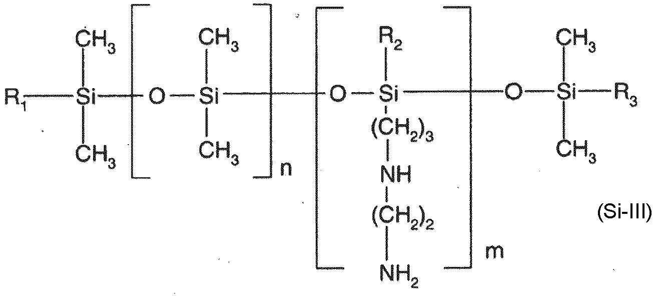 wobei
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere geeignete Verfahren sind gekennzeichnet durch die eines Färbemittels auf den keratinischen Fasern wobei das Färbemittel mindestens aminofunktionelles Silikonpolymer der Formel der Formel (Si-IV) enthält, 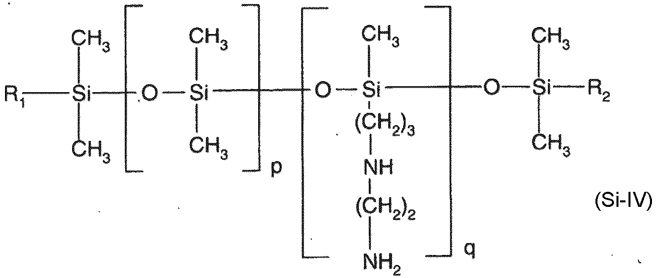 in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-Si(CH₃)₂-Gruppe an eine -[O-Si(CH₃)₂]-Gruppierung gebunden.

Als besonders wirkungsvoll im Hinblick auf die Erzeugung von intensiven Farbresultaten haben sich auch erfindungsgemäßen Verfahren erwiesen, in welchen ein Färbemittel auf den Keratinfasern appliziert wird, welches mindestens ein aminofunktionelles Silikonpolymer der Formel der Formel (Si-V) enthält 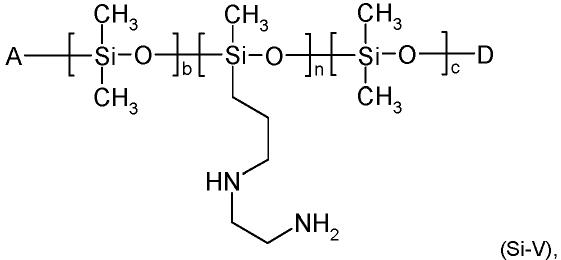 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen,
mit den Maßgaben
- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das applizierte Färbemittel kann weiterhin auch ein oder mehrere verschiedene aminofunktionalisierte Silikonpolymere enthalten, die durch die Formel (Si-VI)

M(RₐQ_{b}SiO_{(4-a-b)/2)x}(R_{c}SiO_{(4-c)/2)y}M (Si-VI)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂ )_{z}NH₂, worin z 1 oder mehr ist. Eine andere mögliche Formel für Z ist -NH(CH₂)_{z}(CH ₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer weiteren Ausführungsform ist ein erfindungsgemäßes Verfahren gekennzeichnet durch die Anwendung eines Färbemittels auf dem keratinischen Fasern, wobei das Färbemittel ein aminofunktionelles Silikonpolymer der Formel (Si-VII)

R'ₐG₃₋ₐ-Si(OSiG ₂)ₙ-(OSiG _{b}R'_{2- b})ₘ-O-SiG₃₋ₐ-R'ₐ (Si-VII),

enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A ,

wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Im Rahmen einer weiteren Ausführungsform ist ein erfindungsgemäßes Verfahren gekennzeichnet durch die Anwendung eines Färbemittels auf dem keratinischen Fasern, wobei das Färbemittel mindestens ein aminofunktionelles Silikonpolymer der Formel (Si-Vlla) enthält, 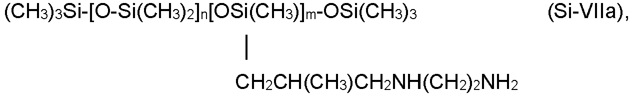 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Färbemittels auf dem keratinischen Fasern, wobei das Färbemittel mindestens ein aminofunktionelles Silikonpolymer der Formel (Si-Vllb) enthält 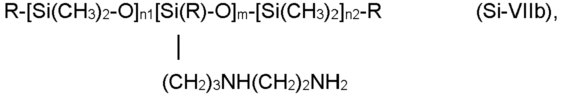 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Färbemittel bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Färbemittel zum Einsatz im erfindungsgemäßen Verfahren geeignet, welche ein spezielles 4-Morpholinomethyl-substituiertes Silikonpolymer enthielten. Dieses aminofunktionalisierte Silikonpolymer umfasst Struktureinheiten der Formeln (SI-VIII) und der Formel (Si-IX)

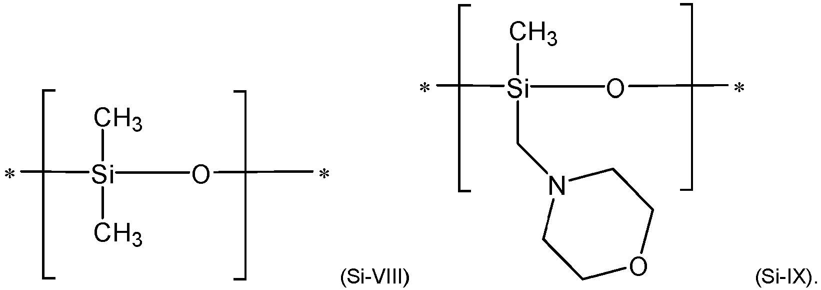

Entsprechende 4-Morpholinomethyl-substituiertes Silikonpolymere werden im folgenden beschrieben.

Ein entsprechendes aminofunktionalisiertes Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist 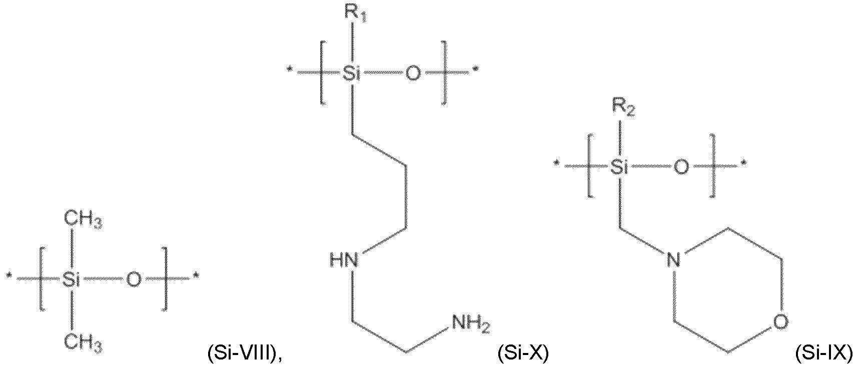 in denen
- R1: für -CH₃, -OH, -OCH₃, -O-CH₂CH₃, -O-CH₂CH₂CH₃, oder -O-CH(CH₃)₂ steht;
- R2: für -CH₃, -OH, oder -OCH₃ steht.

Besonders bevorzugte Färbemittel enthalten mindestens ein 4-morpholinomethyl-substituierten Silikons der Formel (Si-XI) 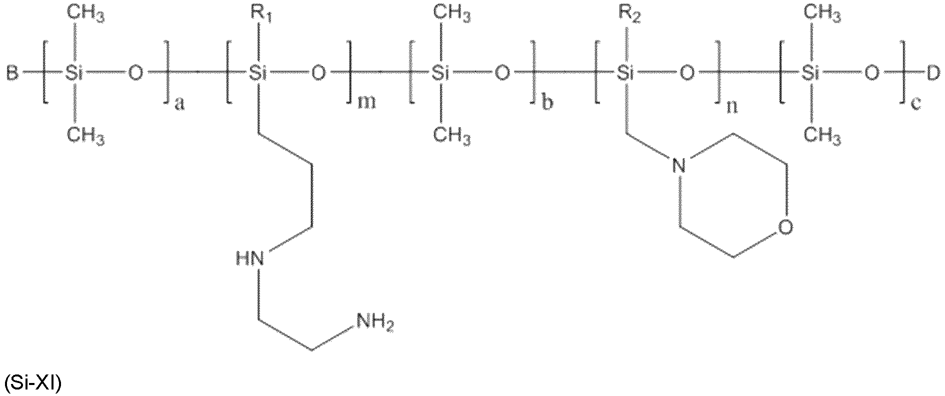 in der
R1 für -CH₃, -OH, -OCH₃, -O-CH₂CH₃, -O-CH₂CH₂CH₃, oder -O-CH(CH₃)₂ steht;
R2 für -CH₃, -OH, oder -OCH₃ steht.
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen
mit den Maßgabe, daß
   - mindestens eine der Bedingungen B = -OH bzw. D = -H erfüllt ist,
   - die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | |
|---|---|
| B = -O-Si(CH₃)₂OH und | D = -Si(CH₃)₃ |
| B = -O-Si(CH₃)₂OH und | D = -Si(CH₃)₂OH |
| B = -O-Si(CH₃)₂OH und | D = -Si(CH₃)₂OCH₃ |
| B = -O-Si(CH₃)₃ | und D = -Si(CH₃)₂OH |
| B = -O-Si(CH₃)₂OCH₃ | und D = -Si(CH₃)₂OH |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Die im Färbeschritt eingesetzten Färbemittel können ein oder mehrere aminofunktionalisierte Silikonpolymere beispielsweise in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthalten. Hierbei sind die Mengenangaben auf die Gesamtmenge aller eingesetzten Aminosilikone bezogen, die zum Gesamtgewicht des Färbemittels in Relation gesetzt wird.

Im Rahmen einer weiteren besonders bevorzugten Ausführungsform ist ein erfindungsgemäßes Verfahren dadurch gekennzeichnet, dass das Färbemittel (F) - bezogen auf das Gesamtgewicht des Färbemittels (F) - ein oder mehrere aminofunktionalisierte Silikonpolymere (f1) in einer Gesamtmenge von 0,1 bis 20 Gew.-%, bevorzugt von 0,2 bis 10 Gew.-%, weiter bevorzugt von 0,3 bis 5 Gew.-%, noch weiter bevorzugt von 0,4 bis 3,5 Gew.-%, und ganz besonders bevorzugt von 0,5 bis 2,0 Gew.-% enthält.

### Pigmente (f2) im Färbemittel (F)

Als zweiten wesentlichen Bestandteil enthält das in Schritt (1) des erfindungsgemäßen Verfahrens angewendete Färbemittel (F) mindestens ein Pigment (f2).

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein. In einer bevorzugten Ausführungsform ist ein erfindungsgemäßes Verfahren dadurch gekennzeichnet, dass das Nachbehandlungsmittel auf Keratinmaterial aufgetragen wird, welches durch Anwendung von mindestens einem anorganischen und/oder organischen Pigment gefärbt wurde.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Färbemittel (F) mindestens ein anorganischen Pigment (f2) enthält, wobei das anorganische Pigment bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Verfahren dadurch gekennzeichnet, dass das Färbemittel (F) mindestens ein Pigment (f2) enthält, das ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das pplizierte Färbemittel auch ein oder mehrere organischen Pigmente enthalten.

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren, dadurch gekennzeichnet, dass das Färbemittel (F) mindestens ein organischen Pigment (f2) enthält, wobei das organische Pigment bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Zur Färbung der Keratinfasern können auch Pigmente mit einer bestimmten Formgebung eingesetzt werden. Beispielsweise kann ein Pigment auf Basis eines lamellaren und/oder eines lentikularen Substratplättchens eingesetzt werden. Weiterhin ist auch die Färbung auf Basis eines Substratplättchens möglich, welches ein Vakuum metallisiertes Pigment umfasst.

Im Rahmen einer weiteren bevorzugten Ausführungsform ist ein erfindungsgemäßes Verfahren dadurch gekennzeichnet, dass das Färbemittel (F) mindestens ein Pigment (f2) enthält, das ausgewählt ist aus der Gruppe der Pigmente auf Basis eines lamellaren Substratplättchens, der Pigmente auf Basis eines lentikularen Substratplättchens und der Vakuum metallisierten Pigmente.

Die Substratplättchen dieses Typs weisen eine durchschnittliche Dicke von höchstens 50 nm, vorzugsweise weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Substratplättchen sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf. Durch die geringe Dicke der Substratplättchen weist das Pigment ein besonders hohes Deckvermögen auf.

Die Substratplättchen sind bevorzugt monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können. Die Substratplättchen sind vorzugsweise homogen aufgebaut, d.h. dass innerhalb der Plättchen kein Konzentrationsgradient auftritt. Insbesondere sind die Substratplättchen nicht schichtartig aufgebaut und weisen keine darin verteilten Teilchen oder Partikel auf.

Die Größe des Substratplättchens kann auf den jeweiligen Anwendungszweck, insbesondere dem gewünschten Effekt auf dem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750, beträgt. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen können aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus Metall(legierung)en aufgebaut.

Als Metall kommt jedes für metallische Glanzpigmente geeignete Metall in Betracht. Derartige Metalle sind unter anderem Eisen und Stahl, sowie alle luft- und wasserbeständigen (Halb)metalle wie beispielsweise Platin, Zink, Chrom, Molybdän und Silicium, sowie deren Legierungen wie Aluminiumbronzen und Messing. Bevorzugte Metalle sind Aluminium, Kupfer, Silber und Gold.

Bevorzugte Substratplättchen sind Aluminiumplättchen und Messingplättchen, wobei Substratplättchen aus Aluminium besonders bevorzugt sind.

Lamellare Substratplättchen zeichnen sich durch einen unregelmäßig strukturierten Rand aus und werden aufgrund ihres Erscheinungsbildes auch als "cornflakes" bezeichnet.

Aufgrund ihrer unregelmäßigen Struktur erzeugen Pigmente auf der Basis von lamellaren Substratplättchen einen hohen Anteil an Streulicht. Außerdem decken die Pigmente auf der Basis von lamellaren Substratplättchen die vorhandene Farbe eines keratinischen Materials nicht vollständig ab und es können beispielsweise Effekte analog zu einer natürlichen Ergrauung erzielt werden.

Lentikulare (= linsenförmige) Substratplättchen weisen einen im Wesentlichen regelmäßigen runden Rand auf und werden aufgrund ihres Erscheinungsbildes auch als "silverdollars" bezeichnet. Aufgrund ihrer regelmäßigen Struktur überwiegt bei Pigmenten auf Basis von lentikularen Substratplättchen der Anteil des reflektierten Lichts.

Vakuum metallisierte Pigmente *(vacuum metallized pigments,* VMP) können beispielsweise durch das Freisetzen von Metallen, Metalllegierungen oder Metalloxiden von entsprechend beschichteten Folien gewonnen werden. Sie zeichnen sich durch eine besonders geringe Dicke der Substratplättchen im Bereich von 5 bis 50 nm und durch eine besonders glatte Oberfläche mit erhöhter Reflektivität aus. Substratplättchen, welche ein im Vakuum metallisiertes Pigment umfassen, werden im Rahmen dieser Anmeldung auch als VMP-Substratplättchen bezeichnet. VMP-Substratplättchen aus Aluminium können beispielsweise durch Freisetzen von Aluminium von metallisierten Folien gewonnen werden.

Die Substratplättchen aus Metall oder Metalllegierung können passiviert sein, beispielsweise durch Eloxieren (Oxidschicht) oder Chromatieren.

Unbeschichtete lamellare, lentikulare und/oder VPM-Substratplättchen, insbesondere solche aus Metall oder Metalllegierung, reflektieren das einfallende Licht in hohem Maße und erzeugen einen Hell-Dunkel-Flop. Diese haben sich zum Einsatz im Färbemittel als besonders bevorzugt erwiesen.

Geeignete Pigmente auf Basis eines lamellaren Substratplättchens umfassen beispielsweise die Pigmente der Reihe VISIONAIRE von Eckart.

Pigmente auf Basis eines lentikularen Substratplättchens sind beispielsweise unter der Bezeichnung Alegrace^{®} Gorgeous von der Firma Schlenk Metallic Pigments GmbH erhältlich.

Pigmente auf Basis eines Substratplättchens, welches ein Vakuum metallisiertes Pigment umfasst, sind beispielsweise unter der Bezeichnung Alegrace^{®} Marvelous oder Alegrace^{®} Aurous von der Firma Schlenk Metallic Pigments GmbH erhältlich.

Aufgrund ihrer ausgezeichneten Licht- und Temperaturbeständigkeit ist die Verwendung der zuvor genannten Pigmente in dem erfindungsgemäßen Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Die Pigmente (f2) werden bevorzugt in bestimmten Mengenbereichen im Färbemittel (F) eingesetzt. Bei dem im erfindungsgemäßen Verfahren zur Färbung eingesetzten Färbemittel können ein oder mehrere Pigmente beispielsweise in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthalten. Hierbei sind die Mengenangaben auf die Gesamtmenge aller eingesetzten Pigmente bezogen, die zum Gesamtgewicht des Färbemittels in Relation gesetzt wird.

In einer weiteren ganz besonders bevorzugten Ausführungsform ist ein erfindungsgemäßes Verfahren dadurch gekennzeichnet, dass das Färbemittel (F) - bezogen auf das Gesamtgewicht des Färbemittels (F) - ein oder mehrere Pigmente (f2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

### Wassergehalt im Färbemittel (F)

Bei dem zuvor beschriebenen Färbemittel (F) handelt es sich um ein anwendungsbereites Mittel, welches auf die keratinischen Fasern appliziert wird. Dieses anwendungsbereite Mittel besitzt bevorzugt einen geringen bis mittleren Wasseranteil. Es hat sich herausgestellt, dass besonders die Färbemittel gut geeignet sind, die - bezogen auf das Gesamtgewicht des Mittels - 0,1 bis 50,0 Gew.-%, bevorzugt 0,5 bis 35,0 Gew.-%, weiter bevorzugt 1,0 bis 20,0 Gew.-% und besonders bevorzugt 1,5 bis 15,0 Gew.-% Wasser enthalten.

In einer weiteren explizit ganz besonders bevorzugten Ausführungsform ist ein Verfahren dadurch gekennzeichnet, dass das Färbemittel (F) - bezogen auf das Gesamtgewicht des Färbemittels (F) - 0,1 bis 50,0 Gew.-%, bevorzugt 0,5 bis 35,0 Gew.-%, weiter bevorzugt 1,0 bis 20,0 Gew.-% und besonders bevorzugt 1,5 bis 15,0 Gew.-% Wasser enthält.

### Kosmetischer Träger des Färbemittels (F)

Aufgrund des zuvor beschriebenen Wassergehalts des Färbemittels, der bevorzugt im mittleren bis geringen Bereich liegt, ist der Hauptbestandteil des kosmetischen Trägers, in dem die Bestandteile (f1) und (f2) des Färbemittels vorliegen, bevorzugt nicht-wässrig. Bei dem kosmetischen Träger handelt es sich bevorzugt um ein Lösungsmittel und/oder um ein Polyethylenglycol. Als geeignete Lösungsmittel können beispielsweise Lösungsmittel aus der Gruppe aus 1,2-Propylenglycol, 1,3-Propylenglycol, 1,2- Butylenglycol, Dipropylenglycol, Ethanol, Isopropanol, Diethylenglycolmonoethylether, Glycerin, Phenoxyethanol und Benzylalkohol eingesetzt werden. Der Einsatz von 1,2-Propylenglycol ist ganz besonders bevorzugt.

Ein ganz besonders bevorzugtes niedermolekulares Polyethylenglycol ist beispielsweise PEG-8. PEG-8 umfasst im Schnitt 8 Ethylenglycol-Einheiten (x1 = 8), besitzt ein mittelres Molgewicht von 400 g/mol und trägt die CAS-Nummer 25322-68-3. PEG-8 wird alternativ auch als PEG 400 bezeichnet und ist beispielsweise von der Firma APS kommerziell erhältlich.

Weitere gut geeignete niedermolekulare Polyethylenglycole sind beispielsweise PEG-6, PEG-7, PEG-9 und PEG-10.

Ein weiteres gut geeignetes Polyethylenglycol ist beispielsweise PEG-32. PEG-32 umfasst 32 Ethylenglycol-Einheiten (x1 = 32), besitzt ein mittleres Molgewicht von 1500 g/mol und trägt die CAS-Nummer 25322-68-3. PEG-32 wird alternativ auch als PEG 1500 bezeichnet und kann zum Beispiel von der Firma Clariant kommerziell erworben werden.

Ein ganz besonders gut geeignetes Polyethylenglycol mit mittlerem Molekulargewicht ist beispielsweise PEG 6000, welches von der Firma National Starch (China) kommerziell erhalten werden kann. Das Molgewicht von PEG 6000 liegt bei 6000 bis 7500 g/mol, dies entspricht einem x3-Wert von 136 bis 171.

### Conditioner (C)

In Schritt (2) des erfindungsgemäßen Verfahrens wird der Conditioner (C) auf den keratinischen Fasern, insbesondere den menschlichen Haaren, angewendet. Der Conditioner ist dadurch gekennzeichnet, dass er mindestens einen Pflegestoff (c1) enthält, der ausgewählt ist aus der Gruppe aus Esterquat-Tensiden, Amidoamin-Tensiden, quartären Imidazolin-Tensiden, Homopolymeren des Diallyldimethylammoniumchlorids, Copolymeren des Diallyldimethylammoniumchlorids, quaternisierten Cellulose-Derivaten, Chitosan und Derivaten des Chitosans.

Bei den Pflegestoffen der Gruppe (c1) handelt es sich um Substanzen, deren pflegende Wirkung im Prinzip bekannt ist und die im Stand der Technik auch als übliche Bestandteile für Conditioner beschrieben sind. Dass ein im Conditioner eingesetzter Pflegestoff das Haargefühl verbessert, mag dem Fachmann daher auf den ersten Blick naheliegend erscheinen.

Die zu dieser Erfindung führenden Arbeiten haben jedoch gezeigt, dass eine größere Anzahl von Pflegestoffen, die im Anschluss an das pigment-basierte Färbeverfahren auf die gefärbten Haare aufgetragen werden, zwar das Haargefühl verbessern, gleichzeitig aber auch die auf den Haaren oder Keratinfasern befindlichen Pigmente wieder abtragen oder rück-dispergieren, so dass die Verbesserung des Haargefühls in diesen Fällen mit einer Verminderung der Farbintensität verbunden ist.

Überraschend wurde nun beobachtet, dass die Anwendung eines Conditioners (C) mit den speziellen Pflegestoffen der Gruppe (c1) im Anschluss an den Färbeschritt das Haargefühl verbessert, ohne dass mit seiner Anwendung auch eine Abschwächung der Farbe verbunden ist. Nach der Anwendung des erfindungsgemäßen Conditioners (C) fühlten sich die Haare weich und natürlich an und waren in derselben hohen Intensität gefärbt wie die Haare, die nicht mit einem Conditioner (C) nachbehandelt wurden.

Es wurde gefunden, dass die Pflegestoffe, mit welchen der oben beschriebene Effekt erzielt werden konnte, zur Gruppe der Esterquat-Tenside, der Amidoamin-Tenside, der quartären Imidazolin-Tenside, der Homopolymere des Diallyldimethylammoniumchlorids, der Copolymere des Diallyldimethylammoniumchlorids, der quaternisierten Cellulose-Derivate, Chitosan und den Derivaten des Chitosans gehören.

Besonders stark ausgeprägt war der Effekt mit Conditionern, die mindestens einen Pflegestoff (c1) ausgewählt ist aus der Gruppe aus Esterquat-Tensiden und/oder Amidoamin-Tensiden enthielten.

Explizit ganz besonders bevorzugt ist daher ein Verfahren zum Färben von keratinischen Fasern, insbesondere menschlichen Haaren, umfassend die
(1) Anwendung eines Färbemittels (F) auf den keratinischen Fasern, welches enthält (f1) mindestens ein aminofunktionalisiertes Silikonpolymer, und (f2) mindestens ein Pigment, und die
(2) Anwendung eines Conditioners (C) auf den keratinischen Fasern, welcher enthält (c1) mindestens einen Pflegestoff ausgewählt aus der Gruppe aus Esterquat-Tensiden und Amidoamin-Tensiden,
wobei es sich bei dem Färbemittel (F) und bei dem Conditioner (C) um verschiedene Formulierungen handelt.

Esterquat-Tenside sind kationische Tenside, die in der Regel auf quartären Triethanol-C₁-C₆-Alkyl-Ammonium-Verbindungen oder quartären Diethanol-Di-C₁-C₆-alkyl-Ammonium-Verbindungen basieren. Die notwendigen langen Kohlenwasserstoffketten sind als Fettsäure-ester angefügt, wobei die Hydroxyl-Gruppen der zuvor beschriebenen Ammonium-Verbindungen mit den Carboxylgruppen von Fettsäuren üblicherweise verestert sind. Zwischen der oder den Hydroxygruppen der Ammonium-Verbindung und der jeweiligen Carboxylgruppe der Fettsäure kann sich auch eine Polyethylen-Einheit oder eine Poly-C₂-C₆-alkylen-Einheit befinden.

Bei ganz besonders gut geeigneten Esterquat-Tensiden handelt es sich um Verbindungen der Formel (T-1) 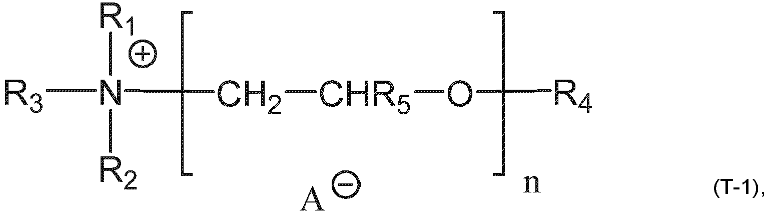 wobei
- R₁, R₂: unabhängig voneinander für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe oder für eine Aryl-C₁-C₆-alkylgruppe stehen,
- R₃: für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe, eine Aryl-C₁-C₆-alkylgruppe oder für eine Gruppierung -X₁-R₆ steht,
- X₁: für eine Gruppe (-CH₂-CHR₇-O)ₘ- steht,
- n: für eine ganze Zahl von 1 bis 200 steht,
- m: für eine ganze Zahl von 1 bis 200 steht
- R₄, R₆: unabhängig voneinander für eine Gruppierung R₈-C(O)- stehen,
- R_{5,} R₇: unabhängig voneinander für Wasserstoff, eine lineare C₁-C₄-Alkylgruppe oder für eine Hydroxygruppe stehen,
- R₈: jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht, und
- A⁻: für ein phyisologisch verträgliches Anion steht.

Die Substituenten R₁ bis R₈ der Verbindung der Formel (I) sind nachfolgend beispielhaft erläutert: Beispiele für eine C₁-C₃₀-Alkylgruppe sind die Gruppen Methyl, Ethyl, Propyl, Isopropyl, n-Butyl, s-Butyl und t-Butyl, n-Pentyl und n-Hexyl, n-Dodecyl, n-Tetradecyl, n-Hexadecyl und n-Octadecyl. Propyl, Ethyl und Methyl sind bevorzugte kurzkettige Alkylreste. n-Dodecyl, n-Tetradecyl, n-Hexadecyl und n-Octadecyl sind bevorzugte langkettigere Alkyl-Reste dieser Gruppe. Beispiele für eine C₂-C₃₀-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine C₁-C₃₀-Hydroxyalkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Arylgruppe sind die Phenyl- und die Napthylgruppe. Bevorzugte Beispiele von Aryl-C₁-C₆-alkylgruppen sind Benzyl, 1-Phenethyl und 2-Phenylethyl.

Im Rahmen einer weiteren ganz besonders bevorzugten Ausführungsform ist ein erfindungsgemäßes Verfahren dadurch gekennzeichnet, dass der Conditioner (C) als Pflegestoff (c1) mindestens einen Esterquat-Tensid der Formal (T-1) enthält, 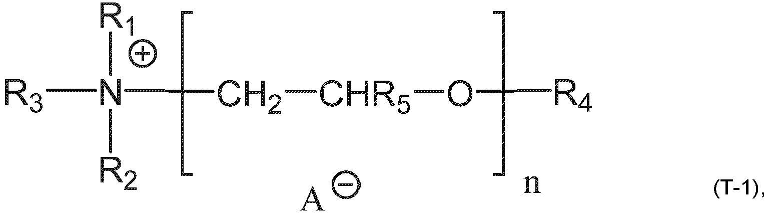 wobei
- R₁, R₂: unabhängig voneinander für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe oder für eine Aryl-C₁-C₆-alkylgruppe stehen,
- R₃: für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe, eine Aryl-C₁-C₆-alkylgruppe oder für eine Gruppierung -X₁-R₆ steht,
- X₁: für eine Gruppe (-CH₂-CHR₇-O)ₘ- steht,
- n: für eine ganze Zahl von 1 bis 200 steht,
- m: für eine ganze Zahl von 1 bis 200 steht
- R₄, R₆: unabhängig voneinander für eine Gruppierung R₈-C(O)- stehen,
- R_{5,} R₇: unabhängig voneinander für Wasserstoff, eine lineare C₁-C₄-Alkylgruppe oder für eine Hydroxygruppe stehen,
- R₈: jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht, und
- A⁻: für ein phyisologisch verträgliches Anion steht.

In den Verbindungen der Formel (T-1) stehen die Reste R₁ und R₂ unabhängig voneinander für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe oder für eine Aryl-C₁-C₆-alkylgruppe.

Bevorzugt stehen die Reste R1 und R2 unabhängig voneinander für einen lineare oder verzweigte C₁-C₃₀-Alkylgruppe oder eine Hydroxy-C₂-C₃₀-alkylgruppe. Besonders bevorzugt steht der Rest R1 für eine C₁-C₆-Alkylgruppe und der Rest R₂ für eine Hydroxy-C₂-C₆-Alkylgruppe. Ganz besonders bevorzugt steht der Rest R₁ für eine Methylgruppe oder eine Ethylgruppe und der Rest R₂ für eine 2-Hydroxyethylgruppe.

In den Verbindungen der Formel (T-1) steht der Rest R₃ für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe, eine Aryl-C₁-C₆-alkylgruppe oder für eine Gruppierung -X₁-R₆.

Bevorzugt steht der Rest R3 für eine C₁-C₃₀-Alkylgruppe wie beispielsweise eine Methylgruppe, eine Ethylgruppe, eine Dodecylgruppe, eine Tetradecylgruppe, eine Hexadecylgruppe oder eine Octadecylgruppe, oder für eine Gruppierung -X₁-R₆ Ganz besonders bevorzugt steht der Rest R3 für eine Gruppierung -X₁-R₆

In der Gruppierung -X₁-R₆ steht der Rest X1 wiederum für eine Gruppe (-CH₂-CHR₇-O)ₘ- , wobei diese Gruppierung so ausgerichtet ist, dass die Methylengruppe -CH2- dem Ammoniumatom in (T-1) benachbart ist und das Sauerstoffatom an den Rest R6 grenzt.

In den Verbindungen der Formel (T-1) steht die Indexzahl n für eine ganze Zahl von 1 bis 200. Beovrzugt steht n für eine ganze Zahl von 1 bis 10. Besonders bevorzugt steht n für die Zahl 1.

In den Verbindungen der Formel (T-1) steht die Indexzahl m für eine ganze Zahl von 1 bis 200. Bevorzugt steht m für eine ganze Zahl von 1 bis 10. Besonders bevorzugt steht m für die Zahl 1.

Die Reste R₄ und R₆ stehen unabhängig voneinander für eine Gruppierung R₈-C(O)-, was bedeutet, dass der Rest R₈ in jeder Gruppierung unabhängig von den anderen Resten R₈ gewählt werden kann.

Die Reste R₅ und R₇ stehen unabhängig voneinander für Wasserstoff, eine lineare C₁-C₄-Alkylgruppe oder für eine Hydroxygruppe. Besonders bevorzugt steht der Rest R₅ für Wasserstoff. Ebenfalls besonders bevorzugt steht der Rest R₇ für Wasserstoff.

Die Verbindungen der Formel (I) können ein oder zwei Gruppierungen R₈-C(O)- enthalten. Sind zwei dieser Gruppierungen enthalten, so kann der Rest R₈ der ersten Gruppierung unabhängig vom Rest R₈ der zweiten Gruppierung gewählt werden.

Der Rest R₈ steht jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe.

Der Rest R8 bildet so jeweils zusammen mit der Carbonylgruppe -C(O)- eine C₆-C₃₀-Alkanoylgruppe bzw. das entsprechend ungesättigte oder Hydroxy-substituierte C₆-C₃₀-Gruppierung aus.

Bevorzugt steht R8 für eine C₁₁-C₂₁-Alkylgruppe, besonders bevorzugt für eine C₁₁-C₁₇-Alkylgruppe.

Explizit ganz besonders bevorzugt stehen die Reste R₄ und R₆ beide für eine Steaoryl-Gruppe.

Der Rest A⁻ steht für ein phyisologisch verträgliches Anion, wobei physiologisch verträglich bedeutet, dass die Substanz der Formel (T-1) mit dem Anion A- unter phyisologischen Bedingungen, d.h. im Rahmen einer kosmetischen Anwendung auf der Haut oder dem Haar des Menschen, in unbedenklicher Weise appliziert werden kann. Phyisologisch vertägliche Anionen sind zum Beispiel das Chlorid, Bromid, Sulfat, Carbonat, Hydrogencarbonat, Methylsulfat oder die Reste anionischen Reste organischer Säuren wie Maleat, Fumarat, Oxalat, Tartrat, Citrat, Lactat oder Acetat. Anionen mit mehreren negativen Ladungen werden hierbei entsprechend stöchiometrisch unter Wahrung der Elektroneutralität der Gesamtverbindung eingesetzt.

Ganz besonders gut geeignete Esterquat-Tenside sind die Verbindungen der Formel (T-2) 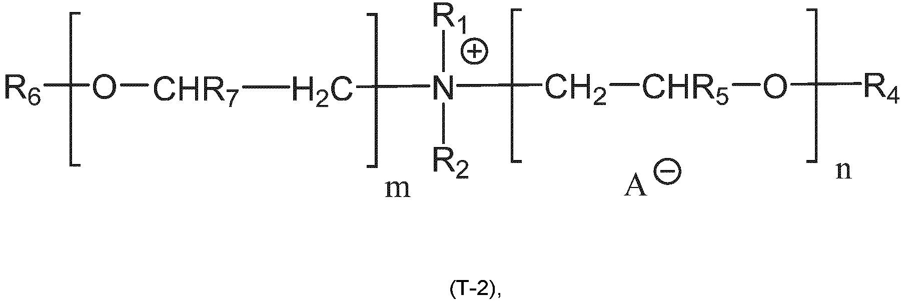 wobei
- R₁,: für eine C₁-C₆-Alkylgruppe steht,
- R2: für eine Hydroxy-C₂-C₆-alkylgruppe steht,
- n: für eine ganze Zahl von 1 bis 200, bevorzugt für die Zahl 1, steht,
- m: für eine ganze Zahl von 1 bis 200, bevorzugt für die Zahl 1, steht,
- R₄, R₆: unabhängig voneinander für eine Gruppierung R₈-C(O)- stehen,
- R_{5,} R₇: unabhängig voneinander für Wasserstoff, eine lineare C₁-C₄-Alkylgruppe oder für eine Hydroxygruppe stehen,
- R₈: jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht, und
- A⁻: für ein phyisologisch verträgliches Anion steht.

Im Rahmen einer weiteren explizit ganz besonders bevorzugten Ausführungsform ist ein erfindungsgemäßes Verfahren dadurch gekennzeichnet, dass der Conditioner (C) als Pflegestoff (c1) mindestens einen Esterquat-Tensid der Formal (T-2) enthält, 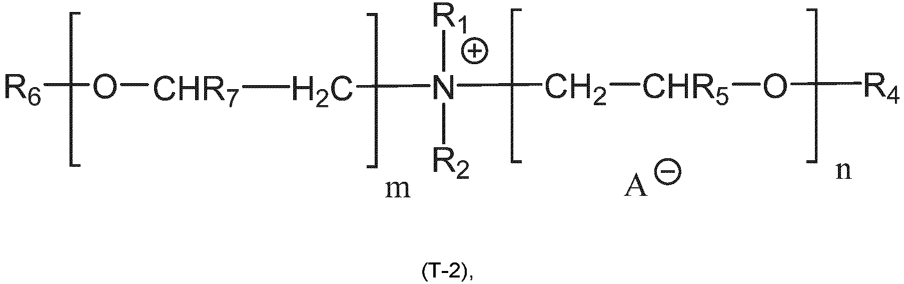 wobei
- R₁,: für eine C₁-C₆-Alkylgruppe steht,
- R2: für eine Hydroxy-C₂-C₆-alkylgruppe steht,
- n: für eine ganze Zahl von 1 bis 200, bevorzugt für die Zahl 1, steht,
- m: für eine ganze Zahl von 1 bis 200, bevorzugt für die Zahl 1, steht,
- R₄, R₆: unabhängig voneinander für eine Gruppierung R₈-C(O)- stehen,
- R_{5,} R₇: unabhängig voneinander für Wasserstoff, eine lineare C₁-C₄-Alkylgruppe oder für eine Hydroxygruppe stehen,
- R₈: jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht, und
- A⁻: für ein phyisologisch verträgliches Anion steht.

Diese Esterquat-Tenside werden beispielsweise unter den Warenzeichen Rewoquat^{®}, Stepantex^{®}, Dehyquart^{®}, Armocare^{®} und Akypoquat^{®} vertrieben. Die Produkte Armocare^{®} VGH-70, Dehyquart^{®} F-75 (INCI-Bezeichnung Distearoylethyl Hydroxyethyl-monium Methosulfate), Dehyquart^{®} C-4046, Dehyquart^{®} L80 T (DICOCOYLETHYL HYDROXYETHYLMONIUM METHOSULFATE, PROPYLENE GLYCOL), Dehyquart^{®} F-30 (CETEARYL ALCOHOL, DIPALMITOYLETHYL HYDROXYETHYLMONIUM METHOSULFATE), Dehyquart^{®} AU-35, Rewoquat^{®} WE18, Rewoquat^{®} WE38 DPG, Stepantex^{®} VS 90 und Akypoquat^{®} 131 sind Beispiele für diese Esterquats.

Die Esterquat-Tenside, insbesondere die zuvor beschriebenen bevorzugten und besonders bevorzugten Vertreter, werden zur optimalen Lösung der erfindungsgemäßen Aufgabenstellung bevorzugt in bestimmten Mengenbereichen im erfindungsgemäßen Conditioner (C) eingesetzt. Besonders gute Ergebnisse wurden erhalten, wenn dass der Conditioner (C) - bezogen auf das Gesamtgewicht des Conditioners (C) - ein oder mehrere Esterquat-Tenside (c1) in einer Gesamtmenge von 0,1 bis 10,0 Gew.-%, bevorzugt 0,2 bis 7,5 Gew.-%, weiter bevorzugt von 0,3 bis 5,0 Gew.-% und ganz besonders bevorzugt von 0,4 bis 2,5 Gew.-% enthielt.

Im Rahmen einer weiteren explizit ganz besonders bevorzugten Ausführungsform ist ein erfindungsgemäßes Verfahren dadurch gekennzeichnet, dass der Conditioner (C) - bezogen auf das Gesamtgewicht des Conditioners (C) - ein oder mehrere Esterquat-Tenside (c1) in einer Gesamtmenge von 0,1 bis 10,0 Gew.-%, bevorzugt 0,2 bis 7,5 Gew.-%, weiter bevorzugt von 0,3 bis 5,0 Gew.-% und ganz besonders bevorzugt von 0,4 bis 2,5 Gew.-% enthält.

Auch Conditioner enthaltend mindestens ein Amidoamin-Tensid haben bei Einsatz im erfindungsgemäßen Färbeverfahren zu gefärbten Haaren mit sehr gutem Haargefühl unter besonders gutem Erhalt der Farbintensität geführt. Aus diesem Grund ist es in einer weiteren Ausführungsform ganz besonders bevorzugt, wenn der Conditioner (C) mindestens ein Amido-Amin Tensid (c1) enthält.

Als Amidoamin-Tenside werden üblicherweise Tenside verstanden, die bei der Reaktion von einer Fettsäure mit einer Mono- oder Diamin-Verbindung erhalten werden. So ist beispielsweise Stearamidopropyl dimethylamin (Lexamine^{®} S-13, Inolex Chemical Company, Philadelphia, PA, USA) das Reaktionsprodukt von Stearinsäure (lineare C18-Fettsäure) mit Dimethylaminopropylamin. Behenamidopropyl dimethylamine (Lexamine^{®} B-13, Inolex Chemical Company, Philadelphia, PA, USA) ist entsprechend das Reaktionsprodukt von Behensäure (lineare C22-Fettsäure) mit Dimethylaminopropylamin. Stearamidoethyl diethylamin (Lexamine^{®} 22, Inolex Chemical Company, Philadelphia, PA, USA) ist das Reaktionsprodukt von Stearinsäure (lineare C18-Fettsäure) mit Diethylaminoethylamin. Kennzeichnend für die Tenside des Amio-amin Strukturtyps ist demach die Amid-Bindung zwischen der Fettsäure und einer ersten Aminogruppe und der mit dieser Amid-bindung über eine gegebenenfalls substituierte Alkylgruppe verbundenen zweiten Aminogruppe.

Besonders starke Effekte konnten beobachtet werden, wenn der Conditioner (C) mindestens ein Amidoamin-Tensid der Formel (T-3) enthielt, 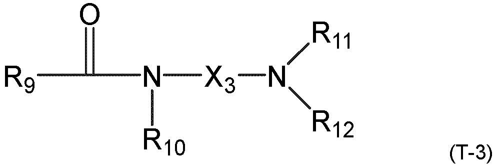 wobei
- R₉: für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht,
- R₁₀: für Wasserstoff, eine C₁-C₄-Alkylgruppe oder für eine Hydroxy-C₁-C₄-Alkylgruppe steht,
- X₃: für eine C₁-C₃₀-Alkylengruppe steht, und
- R₁₁, R₁₂: unabhängig voneinander für Wasserstoff, eine C₁-C₄-Alkylgruppe oder für eine Hydroxy-C₁-C₄-Alkylgruppe stehen.

Im Rahmen einer weiteren ganz besonders bevorzugten Ausführungsform ist ein erfindungsgemäßes Verfahren dadurch gekennzeichnet, dass der Conditioner (C) als Pflegestoff (c1) mindestens ein Amidoamin-Tensid der Formel (T-3) enthält, 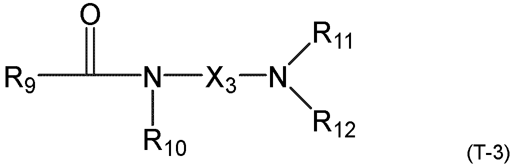 wobei
- R₉: für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht,
- R₁₀: für Wasserstoff, eine C₁-C₄-Alkylgruppe oder für eine Hydroxy-C₁-C₄-Alkylgruppe steht,
- X₃: für eine C₁-C₃₀-Alkylengruppe steht, und
- R₁₁, R₁₂: unabhängig voneinander für Wasserstoff, eine C₁-C₄-Alkylgruppe oder für eine Hydroxy-C₁-C₄-Alkylgruppe stehen.

In den Amidoamin-Tensiden der Formel (T-3) steht der Rest R₉ für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe.

Zusammen mit der Carbonylgruppe, die dem Rest R₉ benachbart ist, bildet die Gruppierung -R₉C(O)-somit C₆-C₃₀-Alkanoylgruppe bzw. die entsprechend ungesättigte oder Hydroxy-substituierte C₆-C₃₀-Gruppierung aus. Besonders bevorzugt steht R₉ für eine C₁₁-C₂₁-Alkylgruppe, besonders bevorzugt für eine C₁₁-C₁₇-Alkylgruppe.

Der Rest R₁₀ steht für Wasserstoff, eine C₁-C₄-Alkylgruppe oder für eine Hydroxy-C₁-C₄-Alkylgruppe. Bevorzugt steht R10 für Wasserstoff.

Der Rest X3 steht für eine C₁-C₃₀-Alkylengruppe. Eine C₁-C₃₀-Alkylengruppe ist eine strukturelle Gruppierung mit Bindungen zu beiden Strickstoffatomen des Amido-amins, so dass diese Alkylengruppe auch als eine divalente oder zweibindige C₁-C₃₀-Alkylengruppe bezeichnet werden, womit gemeint ist, dass jede Gruppierung zwei Bindungen eingehen kann. Beispiele für eine lineare zweiwertige C₁-C₃₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

Besonders bevorzugt steht X3 für eine Ethylengruppe (-CH₂-CH₂-) oder für eine Propylengruppe (-CH₂-CH₂-CH₂-).

Die Reste R₁₁ und R₁₂ stehen unabhängig voneinander für Wasserstoff, eine C₁-C₄-Alkylgruppe oder für eine Hydroxy-C₁-C₄-Alkylgruppe. Besonders bevorzugt stehen beide Reste R11 und R12 für eine Methylgruppe oder für eine Ethylgruppe.

Innerhalb der Gruppe der Amidoamin-Tenside explizit ganz besonders bevorzugt sind die Verbindungen der Formel (T-3), 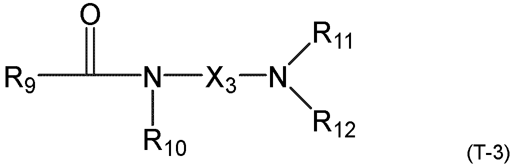 bei welchen
- R₉: für eine lineare oder verzweigte C₅-C₂₉-Alkylgruppe, eine lineare oder verzweigte C₅-C₂₉-Alkenylgruppe oder für eine Hydroxy-C₅-C₂₉-Alkylgruppe steht,
- R₁₀: für Wasserstoff steht,
- X3: für eine Ethylengruppe (-CH₂-CH₂-) oder für eine Propylengruppe (-CH₂-CH₂-CH₂-) steht, und
- R₁₁, R₁₂: beide für eine Methylgruppe oder beide für eine Ethylgruppe stehen.

Im Rahmen einer weiteren ganz besonders bevorzugten Ausführungsform ist ein erfindungsgemäßes Verfahren dadurch gekennzeichnet,_dass der Conditioner (C) als Tensid (c1) mindestens ein Amidoamin der Formel (T-3) enthält,
wobei
- R₉: für eine lineare oder verzweigte C₅-C₂₉-Alkylgruppe, eine lineare oder verzweigte C₅-C₂₉-Alkenylgruppe oder für eine Hydroxy-C₅-C₂₉-Alkylgruppe steht,
- R₁₀: für Wasserstoff steht,
- X3: für eine Ethylengruppe (-CH₂-CH₂-) oder für eine Propylengruppe (-CH₂-CH2-CH₂-) steht, und
- R₁₁, R₁₂: beide für eine Methylgruppe oder beide für eine Ethylgruppe stehen.

Entsprechend ganz besonders bevorzugte Amidoamin-Tenside dieses Typs können ausgewählt werden aus der Gruppe aus Stearamidopropyl-dimethylamin, Behenamidopropyldimethylamin und Stearamidoethyldiethylamin.

Die Amidoamin-Tenside, insbesondere die zuvor beschriebenen bevorzugten und besonders bevorzugten Vertreter, werden zur optimalen Lösung der erfindungsgemäßen Aufgabenstellung bevorzugt in bestimmten Mengenbereichen im erfindungsgemäßen Conditioner (C) eingesetzt. Besonders gute Ergebnisse wurden erhalten, wenn dass der Conditioner (C) - bezogen auf das Gesamtgewicht des Conditioners (C) - ein oder mehrere Amidoamin-Tenside (c1) in einer Gesamtmenge von 0,1 bis 10,0 Gew.-%, bevorzugt 0,2 bis 7,5 Gew.-%, weiter bevorzugt von 0,3 bis 5,0 Gew.-% und ganz besonders bevorzugt von 0,4 bis 2,5 Gew.-% enthielt.

Im Rahmen einer weiteren explizit ganz besonders bevorzugten Ausführungsform ist ein erfindungsgemäßes Verfahren dadurch gekennzeichnet, dass der Conditioner (C) - bezogen auf das Gesamtgewicht des Conditioners (C) - ein oder mehrere Amidoamin-Tenside (c1) in einer Gesamtmenge von 0,1 bis 10,0 Gew.-%, bevorzugt 0,2 bis 7,5 Gew.-%, weiter bevorzugt von 0,3 bis 5,0 Gew.-% und ganz besonders bevorzugt von 0,4 bis 2,5 Gew.-% enthält.

Des weiteren hat es sich als ganz besonders bevorzugt herausgestellt, wenn der Conditioner (C) sowohl ein Esterquat-Tensid als auch ein Amidoamin-Tensid enthält.

Auch Conditioner (C) enthaltend mindestens ein quartäres Imidazolin-Tensid haben sich zur Lösung der erfindungsgemäßen Aufgabenstellung als gut geeignet erwiesen.

Bei quartären Imidazolin-Tensiden handelt es sich um Verbindungen der Formel (T-4) 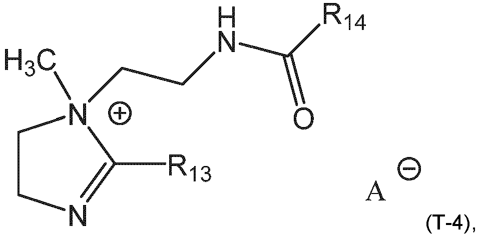 worin
- R13, R14: unabhängig voneinander für einen gesättigten oder ungesättigten, linearen oder verzweigten Kohlenwasserstoffrest mit einer Kettenlänge von 8 bis 30 Kohlenstoffatomen stehen und
- A-: für ein physiologisch verträgliches Anion steht.

Die bevorzugten Verbindungen der Formel (T-5) enthalten für R13 und R14 jeweils den gleichen Kohlenwasserstoffrest. Die Kettenlänge der Reste R13 und R14 ist bevorzugt 12 Kohlenstoff-atome. Besonders bevorzugt sind Verbindungen mit einer Kettenlänge von mindestens 16 Kohlenstoffatomen und ganz besonders bevorzugt mit mindestens 20 Kohlenstoffatomen. Eine ganz besonders bevorzugte Verbindung der Formel I weist eine Kettenlänge von 21 Kohlenstoffatomen auf. Ein Handelsprodukt dieser Kettenlänge ist beispielsweise unter der Bezeichnung Quaternium-91 bekannt. In der Formel (T-4) sheht das Gegenion A- für Methosulfat. Erfindungsgemäß umfasst sind als Gegenionen sind auch die Halogenide wie Chlorid, Fluorid, Bromid, oder auch Phosphate.

Die Imidazoline der Formel (T-4) können in den erfindungsgemäßen Mitteln in Mengen von 0,01 bis 20 Gew.-%, bevorzugt in Mengen von 0,05 bis 10 Gew.-% und ganz besonders bevorzugt in Mengen von 0,1 bis 7,5 Gew.-% enthalten sein. Die allerbesten Ergebnisse werden dabei mit Mengen von 0,1 bis 5 Gew.-% jeweils bezogen auf die Gesamtzusammensetzung des jeweiligen Mittels erhalten.

Auch Conditioner, welche als Pflegestoffe Homopolymere des Diallyldimethylammoniumchlorids, Copolymere des Diallyldimethylammoniumchlorids,quaternisierte Cellulose-Derivaten, Chitosan und/oder Derivate des Chitosans enthielten, haben sich zur Lösung der erfindungsgemäßen Aufgabenstellung als gut geeignet erwiesen.

Ein erfindungsgemäß geeignetes Homopolymer des Diallyldimethylammoniumchlorids ist beipsielsweise Merquat^{®} 100 Poly(dimethyldiallylammoniumchlorid) (ca. 40% Festkörper; INCi-Bezeichnung: Polyquaternium-6, vertrieben von Ondeo Nalco).

Erfindungsgemäß geeignete Copolymere des Diallyldimethylammoniumchlorids sind beispielsweise
- Merquat^{®} 280, ein Copolymer aus Dimethyldiallylammoniumchlorid und Acrylsäure (INCI-Bezeichnung Polyquaternium-22, ca 35 % Aktivstubstanz in Wasser, vertrieben von Ondeo-Nalco),
- Merquat^{®} 281, ein Copolymer aus Dimethyldiallylammoniumchlorid und Acrylsäure (ca. 39-43% Festkörper in Wasser; INCi-Bezeichnung: Polyquaternium-22, vertrieben von Ondeo-Nalco),
- Merquat^{®} 295, ein Copolymer aus Dimethyldiallylammoniumchlorid und Acrylsäure (ca. 35-40%Festkörper in Wasser; INCi-Bezeichnung: Polyquaternium-22, vertriebenOndeo-Nalco),
- Merquat^{®} 550, ein Copolymer aus Dimethyldiallylammoniumchlorid und Acrylami (ca. 8.1-9.1% Aktivsubstanz in Wasser; INCi-Bezeichnung: Polyquaternium-7, vertrieben fon Ondeo-Nalco),
- Merquat^{®} Plus 3330, ein Copolymer aus Dimethyldiallylammoniumchlorid und Acrylsäure und Acrylamid (ca. 9,5% Festkörper in Wasser; INCi-Bezeichnung: Polyquaternium-39, vertrieben von Ondeo-Nalco)
- Merquat^{®} Plus 3330 Dimethyldiallylammoniumchlorid Acrylsäure Acrylamid Terpolymer (ca. 9,5% Festkörper in Wasser; INCi-Bezeichnung: Polyquaternium-39) (Ondeo-Nalco).

Kationische, also quaternisierte Cellulosen sind mit unterschiedlichem Substitutionsgrad, kationischer Ladungsdichte, Stickstoffgehalt und Molekulargewichten auf dem Markt erhältlich. Beispielsweise wird Polyquaternium-67 im Handel unter den Bezeichnungen Polymer^{®} SL oder Polymer^{®} SK (Amerchol) angeboten. Weitere kationische Cellulosen sind unter den Bezeichnungen Polymer JR^{®} 400 (Amerchol, INCi-Bezeichnung Polyquaternium-10) sowie Polymer Quatrisoft^{®} LM-200 (Amerchol, INCi-Bezeichnung Polyquaternium-24). Weitere Handelsprodukte sind die Verbindungen Celquat^{®} H 100 und Celquat^{®} L 200. Schließlich liegt unter der Handelsbezeichnung Mirustyle^{®} CP der Fa. Croda mit Trimonium and Cocodimonium Hydroxyethylcellulose eine weitere derivatisierte Cellulose mit der INCi-Bezeichnung Polyquaternium-72 vor. Polyquaternium-72 kann sowohl in fester Form als auch bereits in wässriger Lösung vorgelöst verwendet werden. Besonders gut geeignete quaternisierte Cellulosen sind Polyquaternium-10, Polyquaternium-24, Polyquaternium-67 und Polyquaternium-72. Innerhalb dieser Gruppe besonders bevorzugt ist Polyquaternium-10.

Chitosane stellen Biopolymere dar und werden zur Gruppe der Hydrokolloide gezählt. Chemisch betrachtet, handelt es sich um partiell deacetylierte Chitine unterschiedlichen Molekulargewichtes.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, die ein durchschnittliches Molekulargewicht von 800.000 bis 1.200.000 Dalton, eine Viskosität nach Brook-field (1 Gew.-o/oig in Glycolsäure) unterhalb von 5000 mPas, einen Deacetylierungsgrad im Bereich von 80 bis 88 % und einem Aschegehalt von weniger als 0,3 Gew.-% aufweisen.

Neben den Chitosanen als typischen kationischen Biopolymeren kommen im Sinne der Erfindung auch kationisch derivatisierte Chitosane (wie z. B. Quaternierungsprodukte) oder alkoxylierte Chitosane in Frage.

Als Chitosanderivate können die erfindungsgemäßen Conditioner (C ) beispielsweise die Neutralisationsprodukte von Chitosan mit mindestens einer Säure enthalten. Die zur Neutralisation genutzte Säure wird bevorzugt ausgewählt aus organischen Säuren, insbesondere aus Ameisensäure, Milchsäure, Pyrrolidoncarbonsäure, Nicotinsäure, Essigsäure, Zitronensäure, Hydroxyisobuttersäure, Hydroxyisovaleriansäure oder Gemischen dieser Säuren. Dabei ist es ganz besonders bevorzugt, die zur Neutralisation genutzte(n) Säure(n) aus Milchsäure, Pyrrolidoncarbonsäure, Nicotinsäure, Hydroxyisobuttersäure, Hydroxyisovaleriansäure oder Gemischen dieser Säuren auszuwählen.

Geeignete Chitosan(derivate) sind beispielsweise unter den Handelsbezeichnungen Hydagen^{®} CMF (1 Gew.-% Aktivsubstanz in wässriger Lösung mit 0.4 Gew.-% Glykolsäure, Molekulargewicht 500000 bis 5000000 g/mol Cognis), Hydagen^{®} HCMF (Chitosan (zu 80 % deacetyliert), Molekulargewicht 50000 bis 1000000 g/mol, Cognis), Kytamer^{®} PC (80 Gew.-% Aktivsubstanz an Chitosan pyrolidoncarboxylat (INCi-Bezeichnung: Chitosan PCA), Amerchol) und Chitolam^{®} NB/101 im Handel frei verfügbar.

Das Chitosan bzw. dessen Derivate kann im Conditioner bei in einer Menge von 0,01 Gew.-% bis 20 Gew.-%, besonders bevorzugt von 0,01 Gew.-% bis 10,0 Gew.-%, ganz besonders bevorzugt von 0,1 Gew.-% bis 1 Gew.-%, jeweils bezogen auf das Gewicht des erfindungsgemäßen Mittels, enthalten.

### pH-Wert des Conditioners (C)

Die pH-Werte des erfindungsgemäßen Conditioners werden bevorzugt auf einen sauren bis neutralen pH-Wert eingestellt. Ganz besonders bevorzugt besitzt das Mittel einen sauren pH-Wert im Bereich von 2,0 bis 6,5, bevorzugt von 2,5 bis 6,0, weiter bevorzugt von 3,0 bis 5,5 und ganz besonders bevorzugt von 4,0 bis 5,0.

Im Rahmen einer weiteren explizit ganz besonders bevorzugten Ausführungsform ist ein erfindungsgemäßes Verfahren dadurch gekennzeichnet, dass der Conditioner (C) - bezogen auf das Gesamtgewicht des Conditioners - mindstens 50 Gew.-% Wasser enthält und einen pH-Wert von 2,0 bis 6,5, bevorzugt von 2,5 bis 6,0, weiter bevorzugt von 3,0 bis 5,5 und ganz besonders bevorzugt von 4,0 bis 5,0 besitzt.

Zur Einstellung des gewünschten pH-Wertes kann der Conditioner mindestens ein Acidifizierungs und/oder Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Als Acidifizierungsmittel kann der Conditioner anorganische Säuren oder organische Säuren wie beispielsweise Zitronensäure, Äpfelsäure, Weinsäure, Milchsäure, Maleinsäure, Malonsäure und/oder Benzoesäure enthalten.

### Färbemittel (F) und Conditioner (C)

Im Rahmen des erfindungsgemäßen Vefahrens werden in den Schritten (1) und (2) ein Färbemittel (F) und ein Conditioner (C) auf den Keratinfasern angewendet, wobei es sich bei dem Färbemittel (F) und bei dem Conditioner (C) um verschiedene Formulierungen handelt.

Färbemittel (F) und Conditioner (C) werden zu verschiedenen Zwecken eingesetzt und enthalten aufgrund ihrer unterschiedlichen Zweckbestimmung auch verschiedene Inhaltsstoffe. Mit dem Färbemittel (F) soll die Färbung der Keratinfasern erreicht werden, so dass das Färbemittel die für die Färbung verantwortlichen Pigmente (f2) enthält. Der Conditioner (C) wird für die spätere Konditionierung der Keratinfasern eingesetzt und enthält daher das oder die zuvor beschriebenen Pflegestoffe der Gruppe (c1).

Aus diesem Grund ist ein erfindungsgemäßes Färbemittel (F) bevorzugt dadurch gekennzeichnet, dass es keine Pflegestoffe der Gruppe (c1) enthält. Das erfindungsgemäße Shampoo (S) ist bevorzugt dadurch gekennzeichnet, dass es keine Pigmente enthält. Auch der Wassergehalt von Färbemittel und Shampoo ist bevorzugt unterschiedlich.

### Wassergehalt im Conditioner (C)

Das Conditioner (C) enthält das oder die aminofunktionalisierten Silikonpolymere (s1) besonders bevorzugt in einem wässrigen kosmetischen Träger. Es hat sich als bevorzugt herausgestellt, wenn der Wasserhalt im Shampoo (S) - bezogen auf das Gesamtgewicht des Shampoos (S) - im Bereich von 50 bis 99 Gew.-%, bevorzugt von 60 bis 97 Gew.-%, weiter bevorzugt von 70 bis 95 Gew.-%, und besonders bevorzugt von 75 bis 93 Gew.-% liegt.

In einer weiteren bevorzugten Ausführungsform ist ein erfindungsgemäßes Verfahren daher dadurch gekennzeichnet, dass das Shampoo (S) - bezogen auf das Gesamtgewicht des Shampoos (S) - 50 bis 99 Gew.-%, bevorzugt von 60 bis 97 Gew.-%, weiter bevorzugt von 70 bis 95 Gew.-%, und besonders bevorzugt von 75 bis 93 Gew.-% Wasser enthält.

### weitere optionale Inhaltsstoffe im Färbemittel (F) und/oder im Shampoo (S)

Zusätzlich zu den bereits beschriebenen erfindungswesentlichen Bestandteilen kann das Färbemittel und/oder der Conditioner auch noch weitere optionale Inhaltsstoffe enthalten, wie beispielsweise Lösungsmittel; Tenside, die von den zuvor beschriebenen Tensiden verschieden sind, anionische, nichtionische, zwitterionische und/oder kationische Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Schritte (1) und (2) des Verfahrens

Das erfindungsgemäße Verfahren umfasst die Anwendung eines Färbemittels (F) auf den keratinischen Fasern in Schritt (1) und die Anwendung eines Conditioners (C) auf den keratinischen Fasern in Schritt (2).

Wie bereits zuvor beschrieben ist es besonders bevorzugt, wenn die Keratinfasern zunächst durch Anwendung des Färbemittels (F) gefärbt werden und danach die Anwendung des Conditioners (C) erfolgt. Der Zeitpunkt, an dem der Conditioners (C) angewendet wird, hängt von den Bedürfnissen das Anwenders ab und kann an dessen Gewohnheiten angepasst werden.

Es ist besonders bevorzugt, den Conditioner (C) auf die frisch gefärbten, noch nassen oder feuchten Keratinfasern aufzutragen, so dass zwischen dem Ausspülen des Färbemittels (F) und der Anwendung des Shampoos (S) ein Zeitraum von nur wenigen Minuten bis einigen Stunden liegt.

In einer weiteren Ausführungsform ganz besonders bevorzugt ist ein Verfahren, umfassend die
(1) Anwendung des Färbemittels (F) auf den keratinischen Fasern in einem ersten Schritt, dann die
(2) Anwendung des Conditioners (C) auf den keratinischen Fasern in einem zweiten Schritt,
wobei zwischen den Schritten (1) und (2) ein Zeitraum von maximal 2 Stunden liegt.

Ein Zeitraum von maximal 2 Stunden zwischen Durchführung der Schritte (1) und (2) kann beispielsweise bedeuten, dass das Färbemittel (F) auf den Keratinfasern angewendet wird, auf die Keratinfasern einwirkt und ausgespült wird, und dann, direkt im Anschluss daran, der Conditioner (C ) auf die Keratinfasern appliziert wird, auf diese einwirkt und ausgespült wird.

In einer weiteren Ausführungsform ganz besonders bevorzugt ist ein Verfahren, umfassend die folgenden Schritte in der angegebenen Reihenfolge:
(1a) Anwendung des Färbemittels (F) auf keratinischen Fasern,
(1b) Einwirken des Färbemittels (F) auf den keratinischen Fasern,
(1c) Ausspülen des Färbemittels,
   dann, direkt im Anschluss an Schritt (1c),
(2a) Anwendung des Conditioners (C) auf den keratinischen Fasern,
(2b) Einwirken des Conditioners (C) und
(2c) Ausspülen des Conditioners.

In Schritt (1a) des erfindungsgemäßen Verfahrens wird ein Färbemittel, welches mindestens ein aminofunktionalisierten Silikonpolymer und mindestens ein Pigment, inbesondere deren zuvor beschriebenen bevorzugte und besonders bevorzugte Vertreter enthält, auf die keratinischen Fasern, insbesondere die Haare, appliziert.

Im darauffolgenden Schritt (1b) wird das zuvor applizierte Färbemittel auf die Haare einwirken gelassen. Ein großer Vorteil des erfindungsgemäßen Färbesystems liegt darin, dass auch in sehr kurzen Zeiträumen nach kurzen Einwirkzeiten ein intensive Farbergebnis erzielt werden kann. Aus diesem Grund ist es von Vorteil, wenn das Färbemittel nach seiner Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In Schritt (1c) wird das Färbemittel (F) wieder aus den Haaren ausgespült oder ausgewaschen, wobei das Auswaschen bevorzugt mit Wasser ohne Zuhilfenahme eines Shampoos und ohne Anwendung einer anderen kosmetischen bzw. tensidischen Formulierung erfolgt.

In Schritt (2a) wird dann der Conditioner (C ) angewendet, wobei die Anwendung bevorzugt direkt im Anschluss an das Ausspülen des Färbemittels und damit auf dem noch nassen oder handtuchfeuchten Haar erfolgt.

Das Einwirken des Conditioners in Schritt (2b) kann beispielsweise für einen Zeitraum von 10 Sekunden bis 5 Minuten erfolgen.

Im Anschluss an Schritt (2b) wird der Conditioner in Schritt (2c) ausgespült. Das Auswaschen des Conditioners erfolgt bevorzugt mit Wasser ohne Zuhilfenahme eines Shampoos und ohne Anwendung einer anderen kosmetischen bzw. tensidischen Formulierung.

### Beispiele

### 1. Formulierungen

Es wurden die folgenden Formulierungen hergestellt (alle Angaben, sofern nichts anderes angegeben ist, in Gew.-%)

| anwendungsbereites Färbemittel (F) | F (Gew.-%) |
|---|---|
| DOWSIL AP-8568 Amino Fluid (Aminosilikon (f1)) | 0,75 |
| Hexamethyldisiloxan | 6,0 |
| Unipure Red LC 3079 (Pigment (f2)) | 0,38 |
| Alegrace Marvelous D 12/77-1 Shiny Silver (10 %ige Dispersion des Pigments) (Pigment (f2)) | 2,30 |
| Wasser | 10,0 |
| PPG-3 Benzyl Ether Myristate | 1,4 |
| Ucon Fluid AP (PPG-14 Butyl Ether) | 1,0 |
| Phenoxyethanol | 0,8 |
| 4-Hydroxyacetophenon | 0,2 |
| Polyethylenglycol (PEG 6000, Molgewicht 6000 g/mol) | 10,0 |
| Polyethylenglycol (PEG 400, Molgewicht 400 g/mol) | ad 100 |

| Conditioner (C) | C (Gew.-%) |
|---|---|
| Isopropylmyristat | 2,00 |
| Dehyquart F 75 T (Distearoylethyl Hydroxyethylmonium Methosulfate (and) Cetearyl Alcohol) | 1,50 |
| Stearamidopropyldimethylamin | 0,80 |
| Cetylpalmitat | 0,70 |
| Cetearylalkohol | 5,20 |
| Behentrimoniumchlorid | 2,0 |
| Guar Hydroxypropyltrimonium Chloride | 0,1 |
| Methylparaben | 0,3 |
| Zitronensäure / Natriumhydroxid | ad pH 4,6 |
| Phenoxyethanol | 0,4 |
| Polydimethylsiloxane 60000 cSt | 0,5 |
| Dehyquart A-CA (Cetrimoniumchlorid (and) water) | 0,50 |
| Parfum | 0,50 |
| Crodarom Black Pearl (Aqua (Water), Glycerin,Hydrolyzed Pearl) | 0,20 |
| Nutrilan Keratin W PP (Hydrolyzed Keratin) | 0,60 |
| Argania Spinosa Shell Powder (Argan Exfoliator 500) | 0,50 |
| Wasser (dest.) | ad 100 |

### 2. Anwendung

Das anwendungsbereite Färbemittel F wurde auf Haarsträhnen (Firma Kerling, Typ "Euronatur-haar weiß" (ENH)) aufgetragen (Flottenverhältnis: 1 g Mittel pro 1 g Haarsträhne) und für drei Minuten einwirken gelassen. Im Anschluss daran wurden die Haarsträhnen gründlich (1 Minute) mit Wasser ausgewaschen. Direkt im Anschluss daran wurde auf die frisch gefärbte, noch feuchte Strähne der Conditioner (C) aufgetragen (Flottenverhältnis 0,5 g Conditioner pro 1 g Haar). Der Conditioner wurde für drei Minuten einwirken gelassen und dann mit Wasser ausgespült.

Eine Vergleichsträhne wurde nach dem Färbeschritt nicht weiter nachbehandelt und getrocknet.

### 3. Bewertung des Griffgefühls

Das Griffgefühl der trockenen Haare wurde durch geschulte Personen bewertet. Die Farbintensität der gefärbten Strähnen wurde visuell unter einer Tageslichtlampe berwertet.

| | Ergebnis Haargefühl | Farbintensität |
|---|---|---|
| Vergleich Anwendung des Färbemittels (F) ohne Nachbehandlung mit Conditioner | einzelne Haarfasern kleben zusammen und lassen sich nicht wie natürliches Haar stylen, klebriges Haargefühl | +++ |
| Erfindung Anwendung des Färbemittels (F) gefolgt von Anwendung des Conditioners (C) | einzelne Haarfasern separiert und gut stylbar, natürliches, weiches Haargefühl | +++ |

| | | |
|---|---|---|
| Farbintensität: + = schwach ++ = mittel +++ = sehr hoch | | |

Durch sukzessive Anwendung des erfindungsgemäßen Färbemittels (F) und des Conditioners (C) konnte das Haargefühl im Vergleich zur Färbung der Haare ohne Nachbehandlung massiv verbessert werden. Das erfindungsgemäße Verfahren ermöglichte intensive Farbergebnissen ohne Verluste im Hinblick auf die Farbintensität.

## Patentansprüche

1. Verfahren zum Färben von keratinischen Fasern, insbesondere menschlichen Haaren, umfassend die
(1) Anwendung eines Färbemittels (F) auf den keratinischen Fasern, welches enthält
(f1) mindestens ein aminofunktionalisiertes Silikonpolymer, und
(f2) mindestens ein Pigment, welches bei 25 °C in Wasser eine Löslichkeit von weniger als 0,05 g/L besitzt, und die
(2) Anwendung eines Conditioners (C) auf den keratinischen Fasern, welcher enthält (c1) mindestens einen Pflegestoff ausgewählt aus der Gruppe aus Esterquat-Tensiden, Amidoamin-Tensiden, quartären Imidazolin-Tensiden, Homopolymeren des Diallyldimethylammoniumchlorids, Copolymeren des Diallyldimethylammonium-chlorids,quaternisierten Cellulose-Derivaten, Chitosan und Derivaten des Chitosans,
wobei es sich bei dem Färbemittel (F) und bei dem Conditioner (C) um verschiedene Formulierungen handelt, und
wobei zwischen den Schritten (1) und (2) ein Zeitraum von maximal 2 Stunden liegt.

2. Verfahren nach Anspruch 1, **dadurch gekennzeichnet, dass** das Färbemittel (F) mindestens ein aminofunktionalisiertes Silikonpolymer (f1) enthält, das mindestens eine Struktureinheit der Formel (Si-Amino) umfasst, 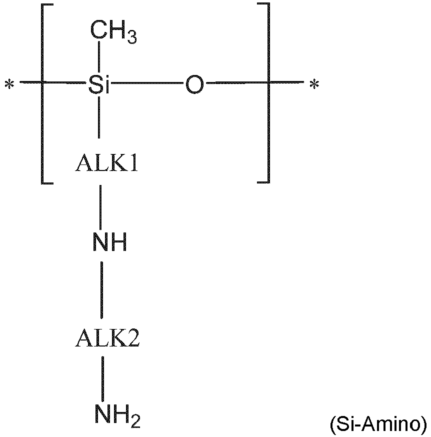 wobei
ALK1 und ALK2 unabhängig voneinander für eine lineare oder verzweigte, zweiwertige C₁-C₂₀-Alkylengruppe stehen.

3. Verfahren nach einem der Ansprüche 1 bis 2, **dadurch gekennzeichnet, dass** das Färbemittel (F) mindestens ein aminofunktionalisiertes Silikonpolymer (f1) enthält, das Struktureinheiten der Formel (Si-I) und der Formel (Si-II) umfasst

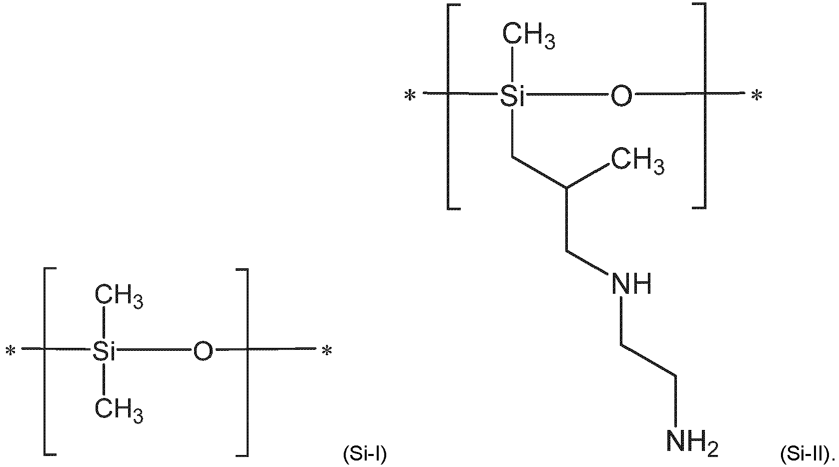

4. Verfahren nach einem der Ansprüche 1 bis 3, **dadurch gekennzeichnet, dass** das Färbemittel (F) - bezogen auf das Gesamtgewicht des Färbemittels (F) - ein oder mehrere aminofunktionalisierte Silikonpolymere (f1) in einer Gesamtmenge von 0,1 bis 20 Gew.-%, bevorzugt von 0,2 bis 10 Gew.-%, weiter bevorzugt von 0,3 bis 5 Gew.-%, noch weiter bevorzugt von 0,4 bis 3,5 Gew.-%, und ganz besonders bevorzugt von 0,5 bis 2,0 Gew.-% enthält.

5. Verfahren nach einem der Ansprüche 1 bis 4, **dadurch gekennzeichnet, dass** das Färbemittel (F) mindestens ein anorganischen Pigment (f2) enthält, wobei das anorganische Pigment bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

6. Verfahren nach einem der Ansprüche 1 bis 5, **dadurch gekennzeichnet, dass** das Färbemittel (F) mindestens ein organisches Pigment (f2) enthält, wobei das organische Pigment bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

7. Verfahren nach einem der Ansprüche 1 bis 6, **dadurch gekennzeichnet, dass** das Färbemittel (F) mindestens ein Pigment (f2) enthält, das ausgewählt ist aus der Gruppe der Pigmente auf Basis eines lamellaren Substratplättchens, der Pigmente auf Basis eines lentikularen Substratplättchens und der Vakuum metallisierten Pigmente.

8. Verfahren nach einem der Ansprüche 1 bis 7, **dadurch gekennzeichnet, dass** das Färbemittel (F) - bezogen auf das Gesamtgewicht des Färbemittels (F) - 0,1 bis 50,0 Gew.-%, bevorzugt 0,5 bis 35,0 Gew.-%, weiter bevorzugt 1,0 bis 20,0 Gew.-% und besonders bevorzugt 1,5 bis 15,0 Gew.-% Wasser enthält.

9. Verfahren nach einem der Ansprüche 1 bis 8, **dadurch gekennzeichnet, dass** der Conditioner (C) als Pflegestoff (c1) mindestens einen Esterquat-Tensid der Formal (T-1) enthält, 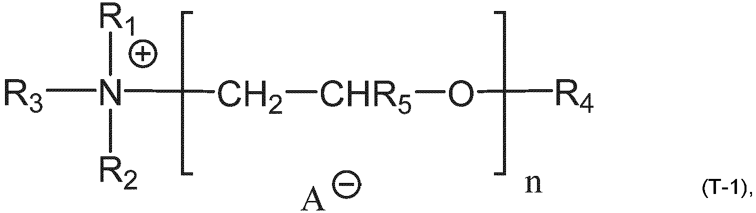 wobei
R₁, R₂ unabhängig voneinander für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe oder für eine Aryl-C₁-C₆-alkylgruppe stehen,
R₃ für eine lineare oder verzweigte C₁-C₃₀-Alkylgruppe, eine lineare oder verzweigte C₂-C₃₀-Alkenylgruppe, eine Hydroxy-C₂-C₃₀-alkylgruppe, eine Arylgruppe, eine Aryl-C₁-C₆-alkylgruppe oder für eine Gruppierung -X₁-R₆ steht,
X₁ für eine Gruppe (-CH₂-CHR₇-O)ₘ- steht,
n für eine ganze Zahl von 1 bis 200 steht,
m für eine ganze Zahl von 1 bis 200 steht
R₄, R₆ unabhängig voneinander für eine Gruppierung R₈-C(O)- stehen,
R_{5,} R₇ unabhängig voneinander für Wasserstoff, eine lineare C₁-C₄-Alkylgruppe oder für eine Hydroxygruppe stehen,
R₈ jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht, und
A⁻ für ein phyisologisch verträgliches Anion steht.

10. Verfahren nach einem der Ansprüche 1 bis 9, **dadurch gekennzeichnet, dass** der Conditioner (C) als Pflegestoff (c1) mindestens einen Esterquat-Tensid der Formal (T-2) enthält, 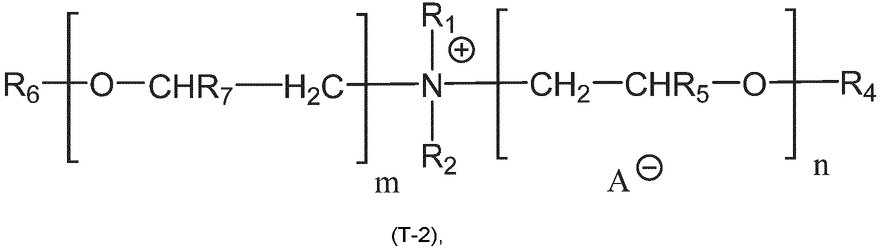 wobei
R₁, für eine C₁-C₆-Alkylgruppe steht,
R2 für eine Hydroxy-C₂-C₆-alkylgruppe steht,
n für eine ganze Zahl von 1 bis 200, bevorzugt für die Zahl 1, steht,
m für eine ganze Zahl von 1 bis 200, bevorzugt für die Zahl 1, steht,
R₄, R₆ unabhängig voneinander für eine Gruppierung R₈-C(O)- stehen,
R_{5,} R₇ unabhängig voneinander für Wasserstoff, eine lineare C₁-C₄-Alkylgruppe oder für eine Hydroxygruppe stehen,
R₈ jeweils unabhängig für eine lineare oder verzweigte C₅-C₂₉-Alkylgruppe, eine lineare oder verzweigte C₅-C₂₉-Alkenylgruppe, oder für eine Hydroxy-C₅-C₂₉-Alkylgruppe steht, und
A⁻ für ein phyisologisch verträgliches Anion steht.

11. Verfahren nach einem der Ansprüche 1 bis 10, **dadurch gekennzeichnet, dass** der Conditioner (C) - bezogen auf das Gesamtgewicht des Conditioners (C) - ein oder mehrere Esterquat-Tenside (c1) in einer Gesamtmenge von 0,1 bis 10,0 Gew.-%, bevorzugt 0,2 bis 7,5 Gew.-%, weiter bevorzugt von 0,3 bis 5,0 Gew.-% und ganz besonders bevorzugt von 0,4 bis 2,5 Gew.-% enthält.

12. Verfahren nach einem der Ansprüche 1 bis 11, **dadurch gekennzeichnet, dass** der Conditioner (C) als Pflegestoff (c1) mindestens ein Amidoamin-Tensid der Formel (T-3) enthält, 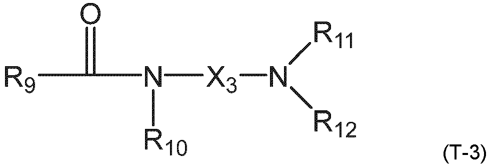 wobei
R₉ für eine lineare oder verzweigte C₅-C₂₉-Alkylgruppe, eine lineare oder verzweigte C₅-C₂₉-Alkenylgruppe oder für eine Hydroxy-C₅-C₂₉-Alkylgruppe steht,
R₁₀ für Wasserstoff, eine C₁-C₄-Alkylgruppe oder für eine Hydroxy-C₁-C₄-Alkylgruppe steht,
X₃ für eine C₁-C₃₀-Alkylengruppe steht, und
R₁₁, R₁₂ unabhängig voneinander für Wasserstoff, eine C₁-C₄-Alkylgruppe oder für eine Hydroxy-C₁-C₄-Alkylgruppe stehen.

13. Verfahren nach einem der Ansprüche 1 bis 12, **dadurch gekennzeichnet, dass** der Conditioner (C) als Pflegestoff (c1) mindestens ein Amidoamin der Formel (T-3) enthält,
wobei
R₉ für eine lineare oder verzweigte C₅-C₂₉-Alkylgruppe, eine lineare oder verzweigte C₅-C₂₉-Alkenylgruppe oder für eine Hydroxy-C₅-C₂₉-Alkylgruppe steht,
R₁₀ für Wasserstoff steht,
X₃ für eine Ethylengruppe (-CH₂-CH₂-) oder für eine Propylengruppe (-CH₂-CH2-CH₂-) steht, und
R₁₁, R₁₂ beide für eine Methylgruppe oder beide für eine Ethylgruppe stehen.

14. Verfahren nach einem der Ansprüche 1 bis 13, **dadurch gekennzeichnet, dass** der Conditioner (C) - bezogen auf das Gesamtgewicht des Conditioners (C) - ein oder mehrere Amidoamin-Tenside (c1) in einer Gesamtmenge von 0,1 bis 10,0 Gew.-%, bevorzugt 0,2 bis 7,5 Gew.-%, weiter bevorzugt von 0,3 bis 5,0 Gew.-% und ganz besonders bevorzugt von 0,4 bis 2,5 Gew.-% enthält.

15. Verfahren nach einem der Ansprüche 1 bis 14, **dadurch gekennzeichnet, dass** der Conditioner (C) - bezogen auf das Gesamtgewicht des Conditioners - mindstens 50 Gew.-% Wasser enthält und einen pH-Wert von 2,0 bis 6,5, bevorzugt von 2,5 bis 6,0, weiter bevorzugt von 3,0 bis 5,5 und ganz besonders bevorzugt von 4,0 bis 5,0 besitzt.

## Claims

1. A method for dyeing keratinous fibers, in particular human hair, comprising
(1) applying a dye (F) to the keratinous fibers, which contains
(f1) at least one amino-functionalized silicone polymer, and
(f2) at least one pigment which has a solubility in water at 25 °C of less than 0.05 g/L, and
(2) applying a conditioner (C) to the keratinous fibers, which contains
(c1) at least one conditioning agent selected from the group consisting of esterquat surfactants, amidoamine surfactants, quaternary imidazoline surfactants, homopolymers of diallyldimethylammonium chloride, copolymers of diallyldimethylammonium chloride, quaternized cellulose derivatives, chitosan and derivatives of chitosan,
wherein the dye (F) and the conditioner (C) are different formulations, and
wherein a time interval of no more than 2 hours elapses between steps (1) and (2).

2. The method according to claim 1, **characterized in that** the dye (F) contains at least one amino-functionalized silicone polymer (f1) comprising at least one structural unit of the formula (Si-Amino), 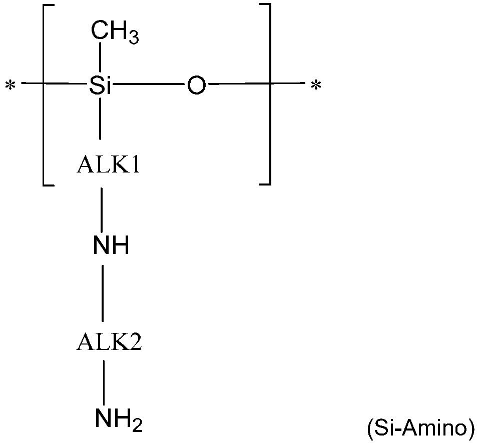 wherein
ALK1 and ALK2 independently represent a linear or branched, divalent C₁-C₂₀ alkylene group.

3. A method according to any one of claims 1 through 2, **characterized in that** the dye (F) contains at least one amino-functionalized silicone polymer (f1) comprising structural units of the formula (Si-I) and the formula (Si-II)

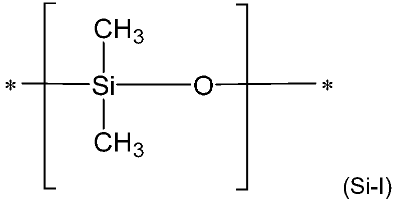

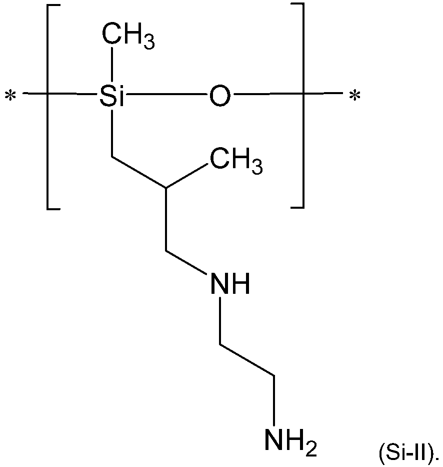

4. A method according to any one of claims 1 through 3, **characterized in that** the coloring agent (F) comprises-based on the total weight of the coloring agent (F)-one or more amino-functionalized silicone polymers (f1) in a total amount of 0.1 to 20 wt. %, preferably from 0.2 to 10 wt.-%, more preferably from 0.3 to 5 wt.-%, even more preferably from 0.4 to 3.5 wt.-%, and most preferably from 0.5 to 2.0 wt.-%.

5. A method according to any one of claims 1 through 4, **characterized in that** the coloring agent (F) contains at least one inorganic pigment (f2), wherein the inorganic pigment is preferably selected from the group consisting of colored metal oxides, metal hydroxides, metal oxide hydrates, silicates, metal sulfides, complex metal cyanides, metal sulfates, bronze pigments, and/or colored pigments based on mica or glimmer that are coated with at least one metal oxide and/or one metal oxychloride.

6. A method according to any one of claims 1 through 5, **characterized in that** the coloring agent (F) contains at least one organic pigment (f2), wherein the organic pigment is preferably selected from the group consisting of carmine, quinacridone, phthalocyanine, sorghum, blue pigments with the Color Index numbers CI 42090, CI 69800, CI 69825, CI 73000, CI 74100, CI 74160, yellow pigments having the Color Index numbers CI 11680, CI 11710, CI 15985, CI 19140, CI 20040, CI 21100, CI 21108, CI 47000, CI 47005; green pigments with the Color Index numbers CI 61565, CI 61570, CI 74260; orange pigments with the Color Index numbers CI 11725, CI 15510, CI 45370, CI 71105, red pigments with the Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, and/or CI 75470.

7. A method according to any one of claims 1 through 6, **characterized in that** the coloring agent (F) contains at least one pigment (f2) selected from the group consisting of pigments based on a lamellar substrate platelet, pigments based on a lenticular substrate platelet, and vacuum-metallized pigments.

8. A method according to any one of claims 1 through 7, **characterized in that** the coloring agent (F)-based on the total weight of the coloring agent (F)-contains 0.1 to 50.0 wt.-%, preferably 0.5 to 35.0 wt.-%, more preferably 1.0 to 20.0 wt.-%, and most preferably 1.5 to 15.0 wt.-% water.

9. A method according to any one of claims 1 through 8, **characterized in that** the conditioner (C) contains, as a conditioning agent (c1), at least one esterquat surfactant of the formula (T-1), 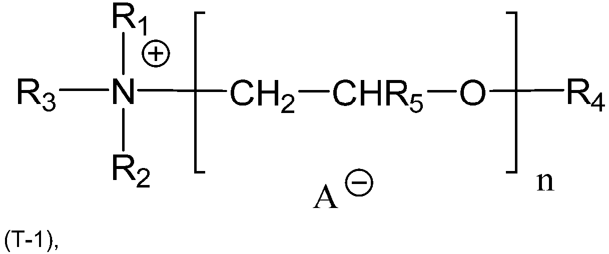 wherein
R₁, R₂ independently of one another represent a linear or branched C₁-C₃₀ alkyl group, a linear or branched C₂-C₃₀ alkenyl group, a hydroxy-C₂-C₃₀ alkyl group, an aryl group, or an aryl-C₁-C₆ alkyl group,
R₃ represents a linear or branched C₁-C₃₀ alkyl group, a linear or branched C₂-C₃₀ alkenyl group, a hydroxy-C₂-C₃₀ alkyl group, an aryl group, an aryl-C₁-C₆ alkyl group, or a group - X₁-R₆,
X₁ represents a group (-CH₂-CHR₇-O)ₘ-,
n represents an integer from 1 to 200,
m represents an integer from 1 to 200,
R₄, R₆ independently of one another represent a group R₈-C(O)-,
R₅, R₇ independently of one another represent hydrogen, a linear C₁-C₄ alkyl group, or a hydroxy group,
R₈ each independently represents a linear or branched C₅-C₂₉ alkyl group, a linear or branched C₅-C₂₉ alkenyl group, or a hydroxy-C₅-C₂₉ alkyl group, and
A⁻ represents a physiologically compatible anion.

10. A method according to any one of claims 1 through 9, **characterized in that** the conditioner (C) contains, as a conditioning agent (c1), at least one esterquat surfactant of the formula (T-2), 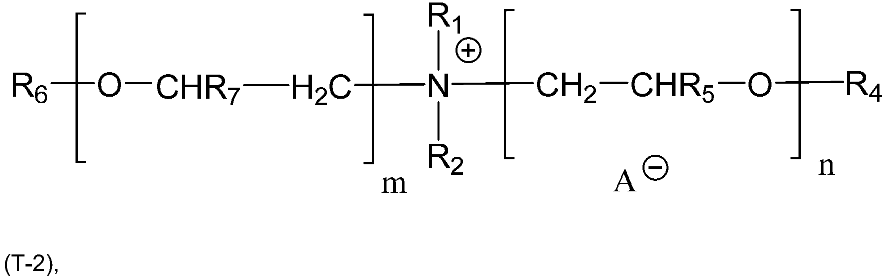 wherein
R₁ represents a C₁-C₆ alkyl group,
R2 represents a hydroxy-C₂-C₆ alkyl group,
n represents an integer from 1 to 200, preferably the number 1,
m represents an integer from 1 to 200, preferably the number 1,
R₄ and R₆ independently represent a group R₈-C(O)-,
R₅ and R₇ independently represent hydrogen, a linear C₁-C₄ alkyl group, or a hydroxy group,
R₈ each independently represents a linear or branched C₅-C₂₉-alkyl group, a linear or branched C₅-C₂₉-alkenyl group, or a hydroxy-C₅-C₂₉-alkyl group, and
A⁻ represents a physiologically compatible anion.

11. A method according to any one of claims 1 through 10, **characterized in that** the conditioner (C)-based on the total weight of the conditioner (C)-contains one or more esterquat surfactants (c1) in a total amount of 0.1 to 10.0 wt.-%, preferably 0.2 to 7.5 wt.-%, more preferably 0.3 to 5.0 wt.-%, and most preferably 0.4 to 2.5 wt.-%.

12. A method according to any one of claims 1 through 11, **characterized in that** the conditioner (C) contains, as a conditioning agent (c1), at least one amidoamine surfactant of the formula (T-3), 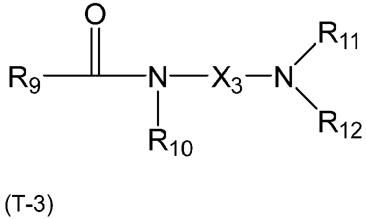 wherein
R₉ represents a linear or branched C₅-C₂₉ alkyl group, a linear or branched C₅-C₂₉ alkenyl group, or a hydroxy-C₅-C₂₉ alkyl group,
R₁₀ represents hydrogen, a C₁-C₄ alkyl group, or a hydroxy-C₁-C₄ alkyl group,
X₃ represents a C₁-C₃₀ alkylene group, and
R₁₁, R₁₂ independently of one another represent hydrogen, a C₁-C₄ alkyl group, or a hydroxy-C₁-C₄ alkyl group.

13. A method according to any one of claims 1 through 12, **characterized in that** the conditioner (C) contains, as a conditioning agent (c1), at least one amidoamine of the formula (T-3),
wherein
R₉ represents a linear or branched C₅-C₂₉-alkyl group, a linear or branched C₅-C₂₉-alkenyl group, or a hydroxy-C₅-C₂₉-alkyl group,
R₁₀ represents hydrogen,
X₃ represents an ethylene group (-CH₂-CH₂-) or a propylene group (-CH₂-CH₂-CH₂-), and
R₁₁, R₁₂ both represent a methyl group or both represent an ethyl group.

14. A method according to any one of claims 1 through 13, **characterized in that** the conditioner (C)-based on the total weight of the conditioner (C)-contains one or more amidoamine surfactants (c1) in a total amount of 0.1 to 10.0 wt.-%, preferably 0.2 to 7.5 wt.-%, more preferably 0.3 to 5.0 wt.-%, and most preferably 0.4 to 2.5 wt.-%.

15. A method according to any one of claims 1 through 14, **characterized in that** the conditioner (C)-based on the total weight of the conditioner-contains at least 50 wt.% water and has a pH of 2.0 to 6.5, preferably 2.5 to 6.0, more preferably 3.0 to 5.5, and most preferably 4.0 to 5.0.

## Revendications

1. Procédé de coloration de fibres kératiniques, en particulier de cheveux humains, comprenant les étapes suivantes :
(1) l'application sur les fibres kératiniques d'un agent colorant (F) qui contient
(f1) au moins un polymère de silicone à fonction amino, et
(f2) au moins un pigment qui présente, à 25 °C, une solubilité dans l'eau inférieure à 0,05 g/L, et l'
(2) application d'un après-shampoing (C) sur les fibres kératiniques, lequel contient
(c1) au moins un agent de soin choisi dans le groupe constitué par les tensioactifs de type esterquat, les tensioactifs de type amidoamine, les tensioactifs imidazolines quaternaires, les homopolymères du chlorure de diallyldiméthylammonium, les copolymères du chlorure de diallyldiméthylammonium, les dérivés de cellulose quaternisés, le chitosane et les dérivés du chitosane, l'agent colorant (F) et l'après-shampoing (C) étant des formulations différentes, et un intervalle de temps maximal de 2 heures s'écoulant entre les étapes (1) et (2).

2. Procédé selon la revendication 1, **caractérisé en ce que** l'agent colorant (F) contient au moins un polymère de silicone à fonction amino (f1) qui comprend au moins un motif structural de formule (Si-amino), 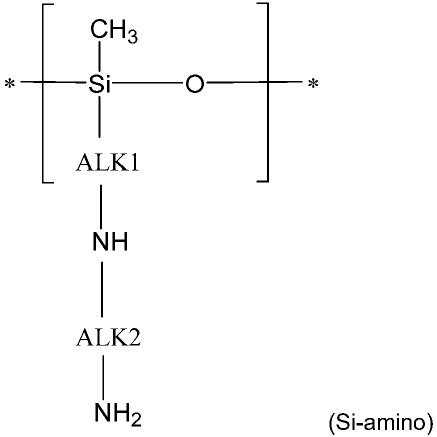 où
ALK1 et ALK2 représentent, indépendamment l'un de l'autre, un groupe alkylène divalent linéaire ou ramifié en C₁ à C₂₀.

3. Procédé selon l'une des revendications 1 à 2, **caractérisé en ce que** l'agent colorant (F) contient au moins un polymère de silicone à fonctionnalité amino (f1) qui comprend des motifs structuraux de formule (Si-I) et de formule (Si-II)

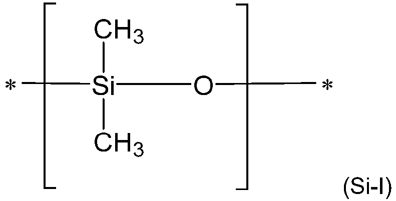

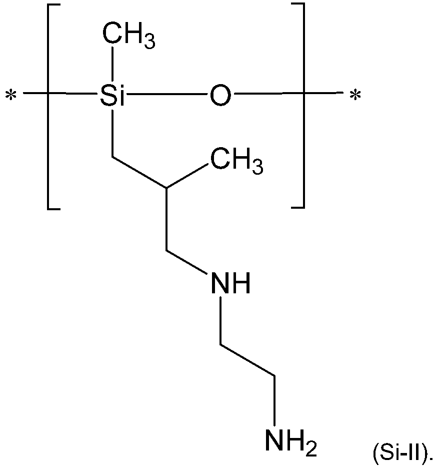

4. Procédé selon l'une des revendications 1 à 3, **caractérisé en ce que** l'agent colorant (F) contient - par rapport au poids total de l'agent colorant (F) - un ou plusieurs polymères de silicone à fonction amino (f1) en une quantité totale de 0,1 à 20 % en poids , de préférence de 0,2 à 10 % en poids, plus préférablement de 0,3 à 5 % en poids, encore plus préférablement de 0,4 à 3,5 % en poids, et tout particulièrement de 0,5 à 2,0 % en poids.

5. Procédé selon l'une des revendications 1 à 4, **caractérisé en ce que** l'agent colorant (F) contient au moins un pigment inorganique (f2), le pigment inorganique étant de préférence choisi dans le groupe des oxydes métalliques colorés, des hydroxydes métalliques, des hydrates d'oxydes métalliques, silicates, des sulfures métalliques, des cyanures métalliques complexes, des sulfates métalliques, des pigments de bronze et/ou parmi les pigments colorés à base de mica ou de mica, qui sont revêtus d'au moins un oxyde métallique et/ou d'un oxychlorure métallique.

6. Procédé selon l'une des revendications 1 à 5, **caractérisé en ce que** l'agent colorant (F) contient au moins un pigment organique (f2), ledit pigment organique étant de préférence choisi parmi le groupe comprenant le carmin, la quinacridone, la phtalocyanine, le sorgho, les pigments bleus portant les numéros d'index de couleur CI 42090, CI 69800, CI 69825, CI 73000, CI 74100, CI 74160, des pigments jaunes portant les numéros d'indice de couleur CI 11680, CI 11710, CI 15985, CI 19140, CI 20040, CI 21100, CI 21108, CI 47000, CI 47005, des pigments verts portant les numéros d'index des couleurs CI 61565, CI 61570, CI 74260, des pigments orange portant les numéros d'index des couleurs CI 11725, CI 15510, CI 45370, CI 71105, des pigments rouges portant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

7. Procédé selon l'une des revendications 1 à 6, **caractérisé en ce que** l'agent colorant (F) contient au moins un pigment (f2) choisi parmi le groupe des pigments à base d'une plaquette de substrat lamellaire, des pigments à base d'une plaquette de substrat lenticulaire et des pigments métallisés sous vide.

8. Procédé selon l'une des revendications 1 à 7, **caractérisé en ce que** l'agent colorant (F) - par rapport au poids total de l'agent colorant (F) - contient 0,1 à 50,0 % en poids, de préférence 0,5 à 35,0 % en poids, mieux encore 1,0 à 20,0 % en poids et de manière particulièrement préférée 1,5 à 15,0 % en poids d'eau.

9. Procédé selon l'une des revendications 1 à 8, **caractérisé en ce que** l'après-shampoing (C) contient, en tant qu'agent de soin (c1), au moins un tensioactif esterquat de formule (T-1), 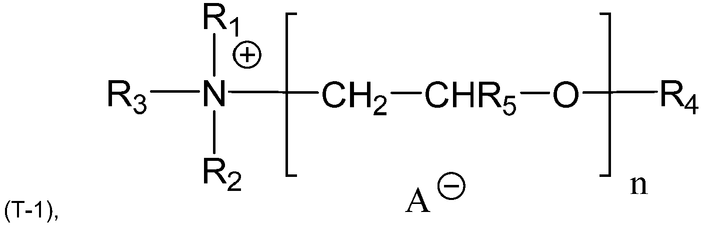 où
R₁, R₂ représentent, indépendamment l'un de l'autre, un groupe alkyle en C₁ à C₃₀ linéaire ou ramifié, un groupe alcényle en C₂ à C₃₀ linéaire ou ramifié, un groupe hydroxyalkyle en C₂ à C₃₀, un groupe aryle ou un groupe arylalkyle en C₁ à C₆,
R₃ représente un groupe alkyle en C₁ à C₃₀ linéaire ou ramifié, un groupe alcényle en C₂ à C₃₀ linéaire ou ramifié, un groupe hydroxyalkyle en C₂ à C₃₀, un groupe aryle, un groupe arylalkyle en C₁ à C₆ ou un groupement -X₁-R₆,
X₁ représente un groupe (-CH₂-CHR₇-O)m-,
n représente un nombre entier compris entre 1 et 200,
m représente un nombre entier compris entre 1 et 200,
R₄, R₆ représentent, indépendamment l'un de l'autre, un groupement R₈-C(O)-,
R₅, R₇ représentent, indépendamment l'un de l'autre, un atome d'hydrogène, un groupe alkyle linéaire en C₁-C₄ ou un groupe hydroxy,
R₈ représente, dans chaque cas indépendamment, un groupe alkyle en C₅ à C₂₉ linéaire ou ramifié, un groupe alcényle en C₅ à C₂₉ linéaire ou ramifié, ou un groupe hydroxyalkyle en C₅ à C₂₉, et
A⁻ représente un anion physiologiquement compatible.

10. Procédé selon l'une des revendications 1 à 9, **caractérisé en ce que** l'après-shampoing (C) contient, en tant qu'agent de soin (c1), au moins un tensioactif esterquat de formule (T-2), 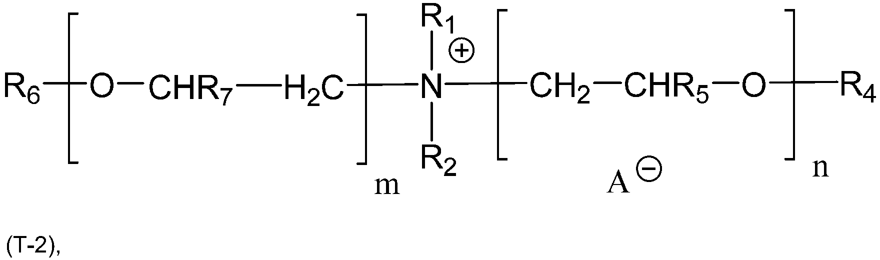 où
R₁, représente un groupe alkyle en C₁-C₆,
R2 représente un groupe hydroxy-alkyle en C₂-C₆,
n représente un nombre entier compris entre 1 et 200, de préférence le nombre 1,
m représente un nombre entier compris entre 1 et 200, de préférence le nombre 1,
R₄ et R₆ représentent, indépendamment l'un de l'autre, un groupement R₈-C(O)-,
R₅ et R₇ représentent, indépendamment l'un de l'autre, un atome d'hydrogène, un groupe alkyle linéaire en C₁ à C₄ ou un groupe hydroxy,
R₈ représente, dans chaque cas indépendamment, un groupe alkyle en C₅ à C₂₉ linéaire ou ramifié, un groupe alcényle en C₅ à C₂₉ linéaire ou ramifié, ou un groupe hydroxyalkyle en C₅ à C₂₉, et
A⁻ représente un anion physiologiquement compatible.

11. Procédé selon l'une des revendications 1 à 10, **caractérisé en ce que** l'après-shampoing (C) - par rapport au poids total de l'après-shampoing
(C) - contient un ou plusieurs tensioactifs de type esterquat (c1) en une quantité totale comprise entre 0,1 et 10,0 % en poids, de préférence entre 0,2 et 7,5 % en poids, de manière encore plus préférée entre 0,3 et 5,0 % en poids et de manière tout particulièrement préférée entre 0,4 et 2,5 % en poids.

12. Procédé selon l'une des revendications 1 à 11, **caractérisé en ce que** l'après-shampoing (C) contient, en tant qu'agent de soin (c1), au moins un tensioactif amidoamine de formule (T-3), 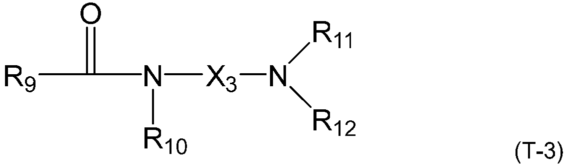 où
R₉ représente un groupe alkyle en C₅ à C₂₉ linéaire ou ramifié, un groupe alcényle en C₅ à C₂₉ linéaire ou ramifié ou un groupe hydroxyalkyle en C₅ à C₂₉,
R₁₀ représente un atome d'hydrogène, un groupe alkyle en C₁ à C₄ ou un groupe hydroxyalkyle en C₁ à C₄,
X₃ représente un groupe alkylène en C₁ à C₃₀, et
R₁₁, R₁₂ représentent, indépendamment l'un de l'autre, un atome d'hydrogène, un groupe alkyle en C₁ à C₄ ou un groupe hydroxyalkyle en C₁ à C₄.

13. Procédé selon l'une des revendications 1 à 12, **caractérisé en ce que** l'après-shampoing (C) contient, en tant qu'agent de soin (c1), au moins une amidoamine de formule (T-3),
dans laquelle
R₉ représente un groupe alkyle en C₅ à C₂₉ linéaire ou ramifié, un groupe alcényle en C₅ à C₂₉ linéaire ou ramifié ou un groupe hydroxyalkyle en C₅ à C₂₉,
R₁₀ représente un atome d'hydrogène,
X₃ représente un groupe éthylène (-CH₂-CH₂-) ou un groupe propylène (-CH₂-CH₂-CH₂-), et
R₁₁, R₁₂ représentent tous deux un groupe méthyle ou tous deux un groupe éthyle.

14. Procédé selon l'une des revendications 1 à 13, **caractérisé en ce que** l'après-shampoing (C) - par rapport au poids total de l'après-shampoing (C) - contient un ou plusieurs tensioactifs amidoamines (c1) en une quantité totale comprise entre 0,1 et 10,0 % en poids, de préférence entre 0,2 et 7,5 % en poids, de manière encore plus préférée entre 0,3 et 5,0 % en poids et de manière tout particulièrement préférée entre 0,4 et 2,5 % en poids.

15. Procédé selon l'une des revendications 1 à 14, **caractérisé en ce que** l'après-shampoing
(C) contient - par rapport au poids total de l'après-shampoing - au moins 50 % en poids d'eau et présente un pH compris entre 2,0 et 6,5, de préférence entre 2,5 et 6,0, de manière encore plus préférentielle entre 3,0 et 5,5 et de manière tout particulièrement préférentielle entre 4,0 et 5,0.
